(19) Europäisches Patentamt
European Patent Office
Office européen des brevets

(11) Numéro de publication : **0 514 274 A1**

(12) # DEMANDE DE BREVET EUROPEEN

(21) Numéro de dépôt : **92401330.3**

(51) Int. Cl.[5] : **C07D 209/44**

(22) Date de dépôt : **15.05.92**

(30) Priorité : **17.05.91 FR 9106036**

(43) Date de publication de la demande : **19.11.92 Bulletin 92/47**

(84) Etats contractants désignés : **PT**

(71) Demandeur : **RHONE-POULENC RORER SA**
**20, avenue Raymond Aron**
**F-92160 Antony (FR)**

(72) Inventeur : **Achard, Daniel**
**26 rue Adrien Tessier**
**F-94320 Thiais (FR)**
Inventeur : **Grisoni, Serge**
**17 rue Babeuf**
**F-94600 Choisy Le Roi (FR)**
Inventeur : **Hanessian, Stephen**
**65 Gables Court, Beacons Fields**
**Quebec, Canada H9W 5H3 (CA)**
Inventeur : **Moutonnier, Claude**
**3 rue Auguste Rodin**
**F-92350 le Plessis Robinson (FR)**
Inventeur : **Peyronel, Jean-François**
**6 Parc d'Ardenay**
**F-91120 Palaiseau (FR)**
Inventeur : **Tabart, Michel**
**Tour Athènes, 75 rue du Javelot**
**F-75013 Paris (FR)**
Inventeur : **Truchon, Alain**
**73 rue Henri Gorjus**
**F-69004 Lyon (FR)**

(74) Mandataire : **Savina, Jacques et al**
**RHONE-POULENC RORER S.A., Direction des Brevets, 20 avenue Raymond Aron**
**F-92160 Antony Cédex (FR)**

(54) **Nouveaux dérivés de perhydroisoindole et leur préparation.**

(57) Nouveaux dérivés de perhydroisoindole de formule générale (I) dans laquelle les radicaux $R_1$ sont identiques et représentent des atomes d'hydrogène ou forment ensemble une liaison, les symboles $R_2$ sont identiques et représentent des radicaux phényle éventuellement substitués par un atome d'halogène ou par un radical méthyle en position 2 ou 3, le symbole $R_3$ représente un atome d'halogène ou un radical hydroxy et le symbole $R_4$ représente un atome d'hydrogène ou simultanément à $R_3$ représente un atome d'halogène, sous leurs formes isomères ou leurs mélanges, et éventuellement leurs sels, et leur préparation.

Les nouveaux dérivés selon l'invention sont des intermédiaires pour la préparation d'antagonistes de la substance P.

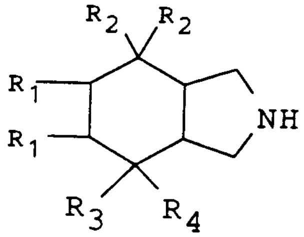

(I)

EP 0 514 274 A1

La présente invention concerne de nouveaux dérivés du perhydroisoindole de formule générale :

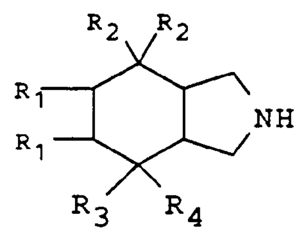

(I)

ainsi que leurs sels, qui sont des intermédiaires pour la préparation de perhydroisoindoles qui antagonisent les effets de la substance P et sont intéressants dans les domaines thérapeutiques où cette substance est connue pour intervenir.

Dans le brevet américain 4 042 707 avaient été décrits des produits dérivés de l'isoindole de formule générale:

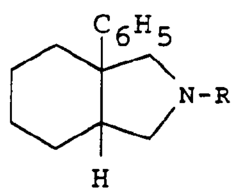

ayant une activité opiacée.

Ces produits n'ont pas d'activité vis à vis de la substance P et ne peuvent pas non plus être utilisés comme intermédiaires pour conduire à de tels produits.

Jusqu'à présent, malgré les recherches mises en oeuvre et malgré l'intérêt suscité [M.R. Hanley, TINS,(5) 139 (1982)], il n'avait pratiquement pas été découvert de produit agissant spécifiquement sur la substance P et ayant une structure non peptidique, c'est pourquoi les dérivés de l'isoindole de formule générale (I) présentent un intérêt considérable.

Dans la formule générale (I) :

- les radicaux $R_1$ sont identiques et représentent des atomes d'hydrogène ou forment ensemble une liaison,
- les symboles $R_2$ sont identiques et représentent des radicaux phényle éventuellement substitués par un atome d'halogène ou par un radical méthyle en position 2 ou 3,
- le symbole $R_3$ représente un atome d'halogène ou un radical hydroxy et
- le symbole $R_4$ représente un atome d'hydrogène ou simultanément à $R_3$ représente un atome d'halogène.
- Lorsque $R_2$ porte un substituant halogène, ou lorsque $R_3$ est un atome d'halogène ,ce dernier peut être choisi parmi le chlore ou le fluor.

Par ailleurs, les produits de formule générale (I) présentent différentes formes stéréoisomères, il est entendu que les dérivés de l'isoindole de forme (3aR,7aR) à l'état pur, ou sous forme de mélange des formes cis (3aRS,7aRS), entrent dans le cadre de la présente invention. Lorsque les radicaux $R_3$ et $R_4$ sont différents, il est également entendu que le substituant $R_3$ peut être en position axiale ou équatoriale et de ce fait que les dérivés R et S ainsi que leurs mélanges entrent aussi dans le cadre de la présente invention.

Selon l'invention le dérivé de l'isoindole de formule générale (I) pour lequel $R_3$ représente un atome d'halogène et $R_4$ représente un atome d'hydrogène ou d'halogène, peut être obtenu par halogénation du dérivé de l'isoindole de formule générale :

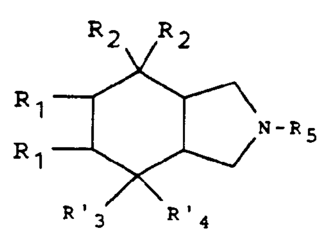

(II)

pour lequel $R_1$ et $R_2$ sont définis comme précédemment, $R'_3$ est un radical hydroxy, $R'_4$ est un atome d'hydrogène si l'on veut obtenir un dérivé monohalogéné, ou $R'_3$ et $R'_4$ forment ensemble un radical oxo si l'on veut obtenir un dérivé dihalogéné, puis élimination du radical protecteur $R_5$.

Le radical protecteur $R_5$ peut être tout groupement protecteur d'amino qui soit compatible avec la réaction et dont la mise en place et l'élimination n'altère pas le reste de la molécule. A titre d'exemple peuvent être cités les groupements alcoyloxycarbonyle, benzyloxycarbonyle, benzyle éventuellement substitués, formyle, chloracétyle, trichloacétyle, trifluoracétyle, vinyloxycarbonyle phénoxycarbonyle, chloro-1 éthoxycarbonyle ou chlorocarbonyle.

Lorsque l'on veut obtenir un produit pour lequel $R_3$ représente un atome de fluor, la réaction s'effectue avantageusement au moyen d'un agent de fluoration comme un fluorure de soufre [trifluorure de morpholino soufre, tétrafluorure de soufre (J. Org. Chem., 40, 3808 (1975)), trifluorure de diéthylamino soufre (Tetrahedron, 44, 2875 (1988)), trifluorure de phényl soufre (J. Am. Chem. Soc., 84, 3058 (1962)], comme l'hexafluoropropyl diéthylamine (brevet Japonais 2 039 546) ou la N-(chloro-2 trifluoro-1,1,2 éthyl) diéthylamine, comme le tétrafluorure de sélenium (J. Am. Chem. Soc., 96,925 (1974) ou comme le tétrafluorophénylphosphorane (Tet. Let., 907 (1973), en opèrant dans un solvant organique tel qu'un solvant chloré (dichlorométhane, dichloréthane par exemple) à une température comprise entre -30 et +30°C. Il est entendu que la mise en oeuvre de l'alcool de configuration (S) conduit au dérivé fluoré de configuration (R) et que la mise en oeuvre de l'alcool de configuration (R) conduit au dérivé fluoré de configuration (S). Il est également possible d'opérer à partir d'un mélange des alcools de configuration (R) et (S) et d'effectuer la séparation au niveau du dérivé de formule générale (I) ainsi obtenu.

Lorsque l'on veut obtenir le dérivé difluoré de formule générale (I), la réaction s'effectue à partir de l'isoindolone de formule générale (II) ($R'_3$ et $R'_4$ forment ensemble un radical oxo), en opérant dans les conditions définies ci-dessus, à une température comprise entre 30°C et la température de reflux du mélange réactionnel.

Lorsque l'on veut obtenir un produit pour lequel $R_3$ représente un atome de chlore, le dérivé chloré de configuration (R) peut être obtenu par traitement de l'alcool (S) par le pentachlorure de phosphore dans les conditions définies par R.J. Cremlyn et coll., J. Chem. Soc., 3794 (1954) ; le dérivé chloré de configuration (S) peut être obtenu par traitement de l'alcool (S) par le chlorure de thionyle dans les conditions citées par R.J. Cremlyn dans la référence mentionnée ci-dessus.

Lorrsque l'on veut obtenir le dérivé dichloré, on offre à partir de la perhydroisoindole de formule générale (II), par traitement par le pentachlorure de phosphore dans les conditions citées ci-dessus.

L'élimination subséquente du radical protecteur $R_5$ s'effectue selon les méthodes habituelles. Notamment, on offre selon les méthodes décrites par T.W. Greene, Protective Groups in Organic Synthesis, A. Wiley - Interscience Publication (1981), ou par Mc Omie, Protective Groups in Organic Chemistry, Plenum Press (1973).

Selon l'invention, le dérivé de l'isoindole de formule générale (I) pour lequel $R_3$ est un atome d'halogène et $R_4$ est un atome d'hydrogène, peut également être obtenu par halogénation d'un dérivé de perhydroisoindole de formule générale :

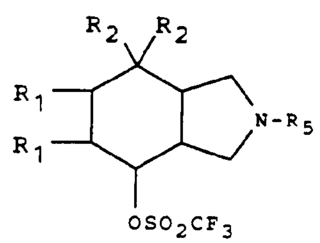 (III)

dans laquelle $R_1$, $R_2$ et $R_5$ sont définis comme précédemment, puis élimination du radical protecteur $R_5$.

L'halogénation s'effectue par un halogénure d'ammonium quaternaire tel que par exemple fluorure de tétrabutylammonium ou par un halogénure alcalin tel que le fluorure de potassium ou le fluorure de césium par exemple, en milieu anhydre, dans un solvant organique tel qu'un éther (tétrahydrofuranne, dioxanne par exemple) un solvant chloré (dichlorométhane par exemple) ou dans un mélange de solvants, à une température comprise entre -30 et 50°C.

Il est entendu que le dérivé sulfonylé de formule générale (III) de configuration (S) conduit à un dérivé halogéné de configuration (R) et que le dérivé sulfonylé de configuration (R) conduit à un dérivé halogéné de configuration (S).

L'élimination du radical $R_5$ s'effectue comme décrit précédemment.

Le dérivé sulfonylé de formule générale (III) peut être obtenu par traiement du dérivé de perhydroisoindole de formule générale (II) pour lequel $R'_3$ est un radical hydroxy et $R'_4$ est un atome d'hydrogène, par un dérivé

réactif de l'acide trifluorométhane sulfonique.

La réaction s'effectue généralement par action de l'anhydride trifluorométhane sulfonique en présence de pyridine, dans un solvant chloré (dichlorométhane par exemple), à une température comprise entre -30 et 20°C.

Selon l'invention, le dérivé de perhydroisoindole de formule générale (I) pour lequel $R_3$ est un radical hydroxy et $R_4$ est un atome d'hydrogène, peut être obtenu par réduction du dérivé de la perhydroisoindolone de formule générale :

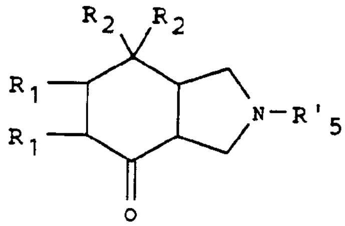

(IV)

dans laquelle $R_1$ et $R_2$ sont définis comme précédemment et $R'_5$ est défini comme $R_5$ ou représente un atome d'hydrogène, suivie de la séparation des isomères axial et équatorial et/ou suivie de l'élimination du radical protecteur lorsque $R'_5$ est autre que l'atome d'hydrogène.

La réduction s'effectue avantageusement au moyen d'un borohydrure alcalin (borohydrure de sodium, tri-s.butylborohydrure de lithium) dans un solvant tel qu'un alcool (méthanol, éthanol par exemple) ou un éther (tétrahydrofuranne) en milieu basique ou par un aluminohydrure (hydrure d'aluminium et de lithium par exemple), à une température comprise entre -20 et 50°C.

L'élimination du radical $R'_5$ s'effectue selon les méthodes connues qui n'affectent pas le reste de la molécule.

Selon l'invention, le dérivé hydroxylé de perhydroisoindole de formule générale (I) dans laquelle $R_3$ est un radical hydroxy et $R_4$ est un atome d'hydrogène peut être aussi obtenu par libération du radical protecteur $R_5$ du dérivé de perhydroisoindole correspondant de formule générale (II) dans laquelle $R'_3$ et $R'_5$ sont définis comme ci-dessus.

L'élimination s'effectue selon les méthodes connues qui n'affectent pas le reste de la molécule, notamment selon les méthodes citées précédemment.

Le dérivé de perhydroisoindole de formule générale (II), ou le dérivé de perhydroisoindole de formule générale (IV) pour lequel $R'_5$ est défini comme $R_5$ peut être préparé par protection de l'amino du derivé correspondant de formule générale :

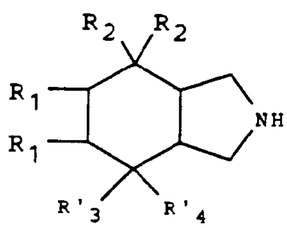

(V)

dans laquelle $R_1$, $R_2$, $R'_3$ et $R'_4$ sont définis comme pour la formule générale (II).

La protection s'effectue selon les méthodes habituelles. Notamment selon les références citées précédemment.

Le dérivés de l'isoindole de formule générale (IV) pour lequel $R'_5$ est un atome d'hydrogène, ou (V) pour lequel $R'_3$ et $R'_4$ forment ensemble un radical oxo, peut être obtenu à partir du dérivé correspondant de formule générale :

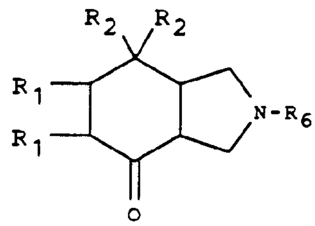

(VI)

dans laquelle $R_1$ et $R_2$ sont définis comme précédemment et $R_6$ représente un radical allyle ou un radical de structure - $CR_aR_bR_c$ dans laquelle $R_a$ et $R_b$ sont des atomes d'hydrogène ou des radicaux phényle éventuellement substitués (par un atome d'halogène, un radical alcoyle, alcoyloxy ou nitro), et $R_c$ est défini comme $R_a$ et $R_b$ ou représente un radical alcoyle ou alcoyloxyalcoyle, l'un au moins de $R_a$, $R_b$ et $R_c$ étant un radical phényle substitué ou non et les radicaux alcoyle contenant 1 à 4 atomes de carbone en chaîne droite ou ramifiée, par élimination du radical $R_6$, par toute méthode connue qui n'affecte pas le reste de la molécule.

Notamment, lorsque $R_1$ est un atome d'hydrogène, et lorsque $R_6$ est autre qu'un radical allyle, le groupement $R_6$ peut être éliminé par hydrogénation catalytique en présence de palladium. Généralement, la réaction s'effectue en milieu acide, dans un solvant tel qu'un alcool (méthanol, éthanol), dans l'eau ou directement dans l'acide acétique ou l'acide formique, à une température comprise entre 20 et 60°C.

Lorsque $R_6$ est un radical benzhydryle ou trityle, l'élimination peut être effectuée par traitement en milieu acide, en offrant à une température comprise entre 0°C et la température de reflux du mélange réactionnel, dans un alcool, dans un éther, dans l'eau ou directement dans l'acide acétique, l'acide formique ou l'acide trifluoroacétique.

Le groupement $R_6$ peut être également éliminé en faisant agir le chloroformiate de vinyle, le chloroformiate de chloro-1 méthyle ou le chloroformiate de phényle, en passant intermédiairement par un produit de formule générale :

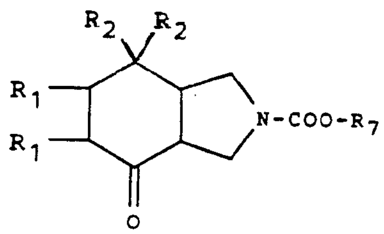

(VII)

dans laquelle $R_1$ et $R_2$ sont définis comme précédemment, et $R_7$ est un radical vinyle, chloro-1 éthyle ou phényle, puis par élimination du radical -$COOR_7$ par traitement acide. L'action du chloroformiate s'effectue généralement dans un solvant organique tel qu'un solvant chloré (dichlorométhane, dichloréthane, chloroforme par exemple), un éther (tétrahydrofuranne, dioxanne par exemple) ou une cétone (acétone par exemple) ou dans un mélange de ces solvants, en offrant à une température comprise entre 20°C et la température de reflux du mélange réactionnel.

L'élimination du radical -$COOR_7$ est effectuée par traitement en milieu acide par exemple par l'acide trifluoroacétique, formique, méthanesulfonique, p.toluènesulfonique, chlorhydrique ou bromhydrique dans un solvant tel qu'un alcool, un éther, un ester, un nitrile, un mélange de ces solvants ou dans l'eau, à une température comprise entre 0°C et la température de reflux du mélange réactionnel.

Dans les conditions d'élimination des radicaux -$COOR_7$ citées précédemment, le dérivé de l'isoindolone de formule générale (IV) ou (V) attendu est-obtenu à l'état de sel de l'acide employé qui peut être mis en oeuvre directement dans l'étape ultérieure.

Le dérivé de l'isoindolone de formule générale (VI) peut être obtenu par réaction de cycloaddition, par action d'un dérivé silylé de formule générale :

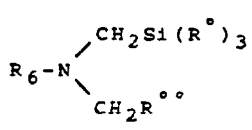

(VIII)

dans laquelle $R_6$ est défini comme précédemment, $(R°)_3$ représente des radicaux alcoyle ou des radicaux alcoyle et phényle et R°° représente un radical alcoyloxy, cyano ou phénylthio, sur le dérivé de la cyclohexènone de formule générale :

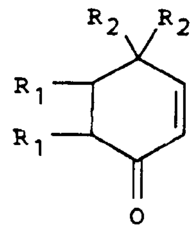

(IX)

dans laquelle $R_1$ et $R_2$ sont définis comme précédemment.

On offre en présence d'une quantité catalytique d'un acide choisi parmi l'acide trifluoroacétique, l'acide acétique, l'acide méthanesulfonique ou les acides cités dans les références mentionnées ci-dessous, dans un solvant organique tel qu'un solvant chloré (dichlorométhane, dichloréthane par exemple), dans un hydrocarbure aromatique, dans un nitrile (acétonitrile) ou dans un éther, à une température comprise entre 0°C et la température de reflux du mélange réactionnel.

Le dérivé silylé de formule générale (VIII) peut être obtenu selon les méthodes décrites par :

- Y. Terao et coll., Chem. Pharm. Bull., 33, 2762 (1985);
- A. Hosomi et coll., Chem. Lett., 1117 (1984)
- A. Padwa et coll., Chem. Ber., 119, 813 (1986) ou
- Tetrahedron, 41, 3529 (1985).

Il est entendu que les dérivés de perhydroisoindole de formule générale (I), (II), (III), (IV), (V), (VI), et (VII) présentent plusieurs formes stéréoisomères. Lorsque l'on veut obtenir un produit de formule générale (I) de forme (3aR,7aR), la séparation des formes isomères peut être mise en oeuvre au niveau du dérivé de formule générale (V) pour lequel $R'_3$ et $R'_4$ forment ensemble un radical oxo. Elle peut aussi être mise en oeuvre au niveau du dérivé de formule générale (I). La séparation s'effectue selon toute méthode connue et compatible avec la molécule.

A titre d'exemple, la séparation peut être effectuée par préparation d'un sel optiquement actif, par action de l'acide L(+) ou D(-) mandélique, ou de l'acide dibenzoyltartrique, puis séparation des isomères par cristallisation. L'isomère recherché est libéré de son sel en milieu basique.

La séparation des isomères axial et équatorial des dérivés hydroxylés ou des dérivés halogénés s'effectue avantageusement au niveau des produits de formule générale (II) ou (V), en offrant par cristallisation et/ou chromatographie. Il est également possible d'opérer au nivau des produits de formule générale (I).

Selon l'invention, les dérivés de l'isoindole de formule générale (I) peuvent être utilisés pour la préparation de dérivés de formule générale :

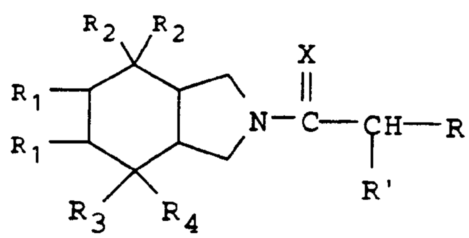

(X)

dans laquelle

- le symbole X représente un atome d'oxygène, ou un radical NH,
- le symbole R représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués [par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle

peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,

- le symbole R′ représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino, et

les symboles $R_1$, $R_2$, $R_3$ et $R_4$ sont définis comme pour la formule générale (I);

les radicaux alcoyle ou acyle cités ci-dessus contennant 1 à 4 atomes de carbone en chaîne droite ou ramifiée;

lorsque R contient un atome d'halogène, ce dernier étant choisi parmi le chlore, le brome, le fluor ou l'iode;

lorsque R représente un radical hétérocyclyle mono ou polycyclique, saturé ou insaturé, celui-ci pouvant être choisi parmi thiényle, furyle, pyridyle, dithiinyle, indolyle, isoindolyle, thiazolyle, isothiazolyle, oxazolyle, imidazolyle, pyrrolyle, triazolyle, thiadiazolyle, quinolyle, isoquinolyle, ou naphtyridinyle;

lorsque R représente phényle substitué par une channe portant un hétérocycle, ce dernier pouvant être choisi parmi pyrrolidinyle, morpholino, pipéridinyle, tétrahydropyridinyle pipérazinyle, ou thiomorpholino.

De plus, lorsque le symbole R′ est autre que l'atome d'hydrogène, la chaîne substituée sur l'isoindole présente un centre chiral, il est entendu que les formes stéréoisomères et leurs mélanges entrent aussi dans la formule générale (X).

Selon l'invention les dérivés du perhydroisoindole de formule générale (X) peuvent être obtenus par action de l'acide de formule générale :

$$R-\underset{\displaystyle R'}{\underset{|}{C}H}-COOH \qquad (XI)$$

ou d'un dérivé réactif de cet acide, dans lequel R et R′ sont définis comme précédemment, sur un dérivé de l'isoindole de formule générale (I) dans laquelle les symboles $R_1$, $R_2$, $R_3$ et $R_4$ sont définis comme précédemment suivie le cas échéant de la transformation de l'amide obtenu en une amidine.

Il est entendu que, les radicaux amino, alcoylamino ou carboxy contenus dans R et/ou R′ sont de préférence préalablement protégés. La protection s'effectue par tout groupement compatible, dont la mise en place et l'élimination n'affectent pas le reste de la molécule. Notamment selon les méthodes décrites par T.W. Greene, par A. Wiley ou par Mc Omie dans les références citées précédemment.

A titre d'exemple,

- les groupements amino ou alcoylamino peuvent être protégés par des radicaux méthoxycarbonyle, éthoxycarbonyle, t.butoxycarbonyle, allyloxycarbonyle, vinyloxycarbonyle, trichloréthoxycarbonyle, trichloracétyle, trifluoracétyle, chloracétyle, trityle, benzhydryle, benzyle, allyle, formyle, acétyle, benzyloxycarbonyle ou ses dérivés substitués;
- les groupements acides peuvent être protégés par des radicaux méthyle, éthyle, t.butyle, benzyle, benzyle substitué ou benzhydryle.

De plus, lorsque R′ représente un radical hydroxy, il est préférable de protéger préalablement ce radical. La protection s'effectue par exemple par un radical acétoxy, trialcoylsilyle, benzyle, ou sous forme d'un carbonate par un radical -COORa dans lequel Ra est un radical alcoyle ou benzyle.

Lorsque l'on effectue la condensation d'un dérivé réactif de l'acide de formule générale (XI), on offre avantageusement au moyen du chlorure d'acide, de l'anhydride, d'un anhydride mixte ou d'un ester réactif dans lequel le reste de l'ester est un radical succinimido, benzotriazolyl-1 éventuellement substitué, nitro-4 phényle, di-nitro-2,4 phényle, pentachlorophényle ou phtalimido.

La réaction s'effectue généralement à une température comprise entre -40 et +40°C, dans un solvant organique tel qu'un solvant chloré (dichlorométhane, dichloréthane, chloroforme par exemple), un éther (tétrahydrofuranne, dioxanne par exemple), un ester (acétate d'éthyle par exemple), un amide (diméthylacétamide, diméthylformamide par exemple), ou une cétone (acétone par exemple) ou dans un mélange de ces solvants, en présence d'un accepteur d'acide tel qu'une base organique azotée comme par exemple la pyridine, la diméthylaminopyridine, la N-méthylmorpholine ou une trialcoylamine (notamment triéthylamine) ou tel qu'un époxyde (oxyde de propylène par exemple). Il est également possible d'opérer en présence d'un agent de

condensation tel qu'un carbodiimide, [par exemple dicyclonexylcarbodiimide ou (diméthylamino-3 propyl)-1 éthyl-3 carboiimide], le NN'-carbonyldiimidazole ou l'éthoxy-2 éthoxycarbonyl-1 dihydro-1,2 quinoléine ou bien en milieu hydroorganique, en présence d'un agent alcalin de condensation comme le bicarbonate de sodium, puis on transforme le cas échéant l'amide obtenu en une amidine telle que définie précédemment.

La transformation de l'amide de formule générale (X) en une amidine pour laquelle X est un radical NH s'effectue en préparant le dérivé de l'isoindolium de formule générale :

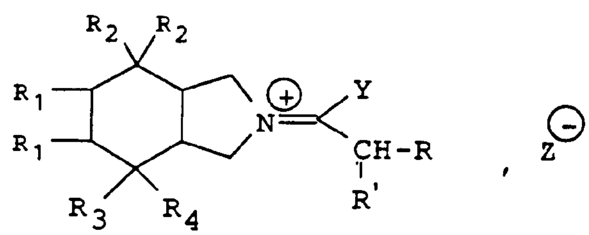

(XII)

dans laquelle R, R', $R_1$, $R_2$ $R_3$ et $R_4$ sont définis comme précédemment, Y représente un atome de chlore, un radical méthoxy ou éthoxy et $Z^-$ représente un ion chlorure, tétrafluoroborate, fluorosulfonate, trifluorométhylsulfonate, méthylsulfate, ou éthylsulfate, puis par action de l'ammoniac sur le dérivé de l'isoindolium.

Il est entendu que, lorsque $R_3$ est hydroxy, Y est autre qu'un atome de chlore.

La préparation du dérivé de l'isoindolium de formule générale (XII) dans laquelle Y est un atome de chlore ou un radical méthoxy ou éthoxy s'effectue par action d'un réactif tel que le phosgène, l'oxychlorure de phosphore, le pentachlorure de phosphore, le chlorure de thionyle, le chlorure d'oxalyle, le chloroformiate de trichlorométhyle, le tétrafluoroborate de triéthyl (ou de triméthyl) oxonium, le triflate de méthyle (ou d'éthyle), le fluorosulfonate de méthyle (ou d'éthyle) ou le sulfate de méthyle (ou d'éthyle). La réaction s'effectue dans un solvant chloré (dichlorométhane, dichloréthane par exemple) ou dans un hydrocarbure aromatique (toluène par exemple), à une température comprise entre 0°C et la température de reflux du mélange réactionnel. L'action de l'ammoniac sur le dérivé de formule générale (XII) s'effectue dans un solvant organique anhydre tel qu'un solvant chloré (dichlorométhane, dichloréthane par exemple), dans un mélange alcool-solvant chloré, dans un éther (tétrahydrofurane par exemple), dans un ester (par exemple acétate d'éthyle), dans un solvant aromatique (toluène par exemple) ou dans un mélange de ces solvants, à une température comprise entre -20°C et la température de reflux du mélange réactionnel.

Il n'est pas indispensable d'avoir isolé le dérivé de l'isoindolium de formule générale (XII) pour le mettre en oeuvre dans cette réaction.

Les dérivés de l'isoindole de formule générale (X) pour lesquels X est un radical imino peuvent également être obtenus à partir du dérivé de l'isoindole selon l'invention, par action d'un produit de formule générale :

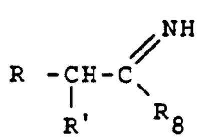

(XIII)

éventuellement à l'état de sel, dans laquelle R et R' sont définis comme précédemment et $R_8$ représente un radical alcoyloxy contenant 1 à 4 atomes de carbone en chaîne droite ou ramifiée ou un radical méthylthio, éthylthio, benzylthio ou alcoyloxycarbonylméthylthio.

La réaction est mise en oeuvre au moyen du dérivé de formule générale (XIII) éventuellement préparé in situ, dans un solvant organique tel qu'un solvant chloré (dichlorométhane, dichloréthane par exemple), un éther (tétrahydrofuranne par exemple), un hydrocarbure aromatique (toluène par exemple) ou un nitrile par exemple l'acétonitrile à une température comprise entre 0°C et la température de reflux du mélange réactionnel.

Il est entendu que, dans l'éventualité où les radicaux R et/ou R' du produit de formule générale (XIII) portent des substituants pouvant interférer avec la réaction, ces derniers doivent être préalablement protégés.

Les acides de formule générale (XI) sont préparés selon les méthodes connues ou décrites ci-après dans les exemples, ou par analogie avec ces méthodes.

Les nouveaux dérivés de l'isoindole de formule générale (I) et les produits de formule générale (X) auxquels ils conduisent peuvent être purifiés le cas échéant par des méthodes physiques telles que la cristallisation ou la chromatographie.

Le cas échéant, les nouveaux dérivés de formule générale (I), ainsi que les produits de formule générale (X) auquels ils conduisent pour lesquels les symboles R et/ou R′ contiennent des substituants amino ou alcoylamino et/ou X représente un radical NH, peuvent être transformés en sels d'addition avec les acides. Comme exemples de sels d'addition avec des acides pharmaceutiquement acceptables, peuvent être cités les sels formés avec les acides minéraux (chlorhydrates, bromhydrates, sulfates, nitrates, phosphates) ou avec les acides organiques (succinates, fumarates, tartrates, acétates, propionates, maléates, citrates, méthanesulfonates, p.toluènesulfonates, iséthionates, ou avec des dérivés de substitution de ces composés).

Les nouveaux dérivés de l'isoindole de formule générale (X) antagonisent les effets de la substance P et peuvent ainsi trouver une application dans les domaines de l'analgésie, de l'inflammation de l'asthme, des allergies, sur le système nerveux central, sur le système cardiovasculaire, comme antispasmodique, ou sur le système immunitaire, ainsi que dans le domaine de la stimulation des secrétions lachrymales.

En effet, les produits selon l'invention manifestent une affinité pour les récepteurs à substance P à des doses comprises entre 5 et 2000 nM selon la technique décrite par C.M. Lee et coll., Mol. Pharmacol., 23, 563-69 (1983).

Il a de plus été démontré qu'il s'agit d'un effet antagoniste de la substance P, au moyen de différents produits. Dans la technique décrite par S, Rosell et coll., Substance P, Ed. by US Von Euler and B. Pernow, Raven Press, New-York (1977), pages 83 à 88, les produits étudiés se sont montrés actifs à des doses comprises entre 20 et 1000 nM.

La substance P est connue pour être impliquée dans un certain nombre de domaines pathologigues :

- Agonists and antagonists of substance P, A.S. Dutta Drugs of the futur, 12 (8), 782 (1987);
- Substance P and pain : an updating, J.L. Henry, TINS, 3(4), 97 (1980);
- Substance P in inflammatory reactions and pain, S. Rosell, Actual. Chim. Ther., 12ème série, 249 (1985)
- Effects of Neuropeptides on Production of Inflammatory Cytokines by Human Monocytes, M. Lotz et coll., Science, 241, 1218 (1988).
- Neuropeptides and the pathogenesis of allergy, Allergy, 42, 1 à 11 (1987);
- Substance P in Human Essential Hypertension, J. Cardiovascular Pharmacology, 10 (suppl. 12), 5172 (1987).

Par ailleurs, les dérivés de l'isoindole de formule générale (X) ne présentent pas de toxicité, ils se sont montrés atoxiques chez la souris par voie sous cutanée à la dose de de 40 mg/kg ou par voie orale à la dose de 100 mg/kg.

D'un intérêt particulier sont les dérivés de perhydroisoindole de formule générale (I) pour lesquels :

- les radicaux $R_1$ sont des atomes d'hydrogène,
- les symboles $R_2$ sont identiques et représentent des radicaux phényle,
- le symbole $R_3$ représente un atome de fluor ou de chlore ou un radical hydroxy et
- le symbole $R_4$ représente un atome d'hydrogène ou simultanément à $R_3$ représente un atome de fluor.

Et parmi ces produits plus spécialement intéressants sont les produits suivants :

- diphényl-7,7 perhydroisoindolol-4,
- diphényl-4,4 fluoro-7 perhydroisoindole,
- diphényl-4, 4 difluoro-7, 7 perhydroisoindole,
- chloro-7 diphényl-4, 4 perhydroisoindole,

sous leurs formes stéréoisomères ainsi que leurs mélanges et leurs sels.

Les exemples suivants donnés à titre non limitatif illustrent la présente invention.

Dans les exemples gui suivent, il est entendu que, sauf mention spéciale, les spectres de RMN du proton ont été faits à 250 MHz dans le diméthylsulfoxyde; les déplacements chimiques sont exprimés en ppm.

## Exemple 1

A une solution de 100 g de chlorhydrate de diphényl-7,7 perhydroisoindolone-4-(3aR,7aR) dans 1000 $cm^3$ de méthanol absolu refroidie à 5°C on ajoute en 90 minutes une solution de 7,18 g de borohydrure de sodium dans 500 $cm^3$ de méthanol additionnée de 20 gouttes de solution concentrée de soude. Après agitation pendant 2,5 heures entre 5 et 10°C, les cristaux formés sont essorés et repris par 900 $cm^3$ d'eau et 1000 $cm^3$ d'éther éthylique. La solution est filtrée et alcalinisée par 15 $cm^3$ de solution de soude 4N puis agitée 2 heures à 5°C. Les cristaux formés sont essorés, lavés à l'éther éthylique et séchés pour donner 28,8 g de diphényl-7,7 perhydroisoindolol-4-(3aR,7S,7aR) sous la forme de cristaux blancs fondant à 205°C, $[\alpha]_D^{20} = -230°$ (c=1, $CHCl_3$).

Le chlorhydrate de diphényl-7,7 perhydroisoindolone-4-(3aR,7aR) peut être préparé de la manière suivante :

A une suspension de 200 g de chlorhydrate de diphényl-7,7 perhydroisoindolone-4 (3aRS,7aRS) dans 2000 $cm^3$ d'acétate d'éthyle, on ajoute lentement, sous agitation, 500 $cm^3$ de soude aqueuse 4N ; l'agitation

est poursuivie jusqu'à dissolution du produit de départ. La solution organique est lavée par 250 $cm^3$ d'eau distillée, par 250 $cm^3$ d'une solution aqueuse saturée en chlorure de sodium, séchée sur sulfate de magnésium et filtrée. A la solution ainsi obtenue, on ajoute, sous agitation, une solution de 92,8 g d'acide L-(+) mandélique dans 1000 $cm^3$ d'acétate d'éthyle ; après 4 heures d'agitation, les cristaux obtenus sont éssorés, lavés par 250 $cm^3$ d'acétate d'éthyle (2 fois) et séchés. Les cristaux sont repris à 2000 $cm^3$ d'eau distillée ; le mélange est chauffé au reflux, sous agitation, pendant 15 minutes ; les cristaux insolubles sont essorés, lavés par 100 $cm^3$ d'eau distillée (2 fois ) et séchés. Ils sont recristallisés dans un mélange de 1100 $cm^3$ d'acétonitrile et de 500 $cm^3$ d'eau distillée; les cristaux obtenus sont essorés, lavés par 40 $cm^3$ d'acétonitrile (3 fois) et séchés. On obtient 80 g de (L)-mandélate de diphényl-7,7 perhydroisoindolone-4 (3aR,7aR); $[\alpha]]_D^{20}$ = -164° (c=1, méthanol).

A 80 g de (L)-mandélate de diphényl-7,7 perhydroisoindolone-4 (3aR,7aR), on ajoute 400 $cm^3$ de soude aqueuse 1N et 600 $cm^3$ d'acétate d'éthyle ; le mélange est agité à température ambiante jusqu'à dissolution du produit de départ ; la solution organique est lavée par 250 $cm^3$ d'eau distillée, par 250 $cm^3$ d'une solution aqueuse saturée en chlorure de sodium, séchée sur sulfate de magnésium et filtrée ; elle est acidifiée, sous agitation, par addition de 30 $cm^3$ d'acide chlorhydrique 9N ; les cristaux obtenus sont essorés, lavés par 50 $cm^3$d'acétate d'éthyle (2 fois), par 50 $cm^3$ d'oxyde d'isopropyle et séchés. On obtient 52,3 g de chlorhydrate de diphényl-7,7 perhydroisoindolone -4 (3aR,7aR), fondant à 270°C avec décomposition; $[\alpha]_D^{20}$= -282° (c=0,5, méthanol).

Le chlorhydrate de diphényl-7,7 perhydroisoindolone-4-(3aRS,7aRS) peut être préparé selon la méthode suivante :

A 15 g de palladium sur charbon à 10%, on ajoute 150 g de benzyl-2 diphényl-7, 7 perhydroisoindolone-4 (3aRS, 7aRS), 1500 $cm^3$ de méthanol et 450 $cm^3$ d'acide chlorhydrique 1N ; le mélange réactionnel est hydrogéné, sous agitation, à température ambiante et sous pression atmosphérique. Après 5 heures de réaction le volume théorique d'hydrogène a été absorbé ; le mélange réactionnel est filtré, puis concentré à sec sous pression réduite (2,7 kPa) ; le résidu est cristallisé dans 200 $cm^3$ d'éthanol ; les cristaux obtenus sont essorés, lavés par 50 $cm^3$d'éthanol et séchés. On obtient 110 g de chlorhydrate de diphényl-7,7 perhydro-isoindolone-4-(3aRS,7aRS) fondant à 270°C avec décomposition.

Spectre de RMN du proton :

2,03 (Mt, 1H, 1H du H en 5 ou 6) ; 2,3 (Mt, 1H, 1H du - H en 5 ou 6) ; 2,48 (DD, en partie masqué, 1H du $-CH_2-$ en 1) ; 2,69 (DD, 1H, 1H du $-CH_2-$ en 1); 2,8 (Mt, 2H, $-CH_2-$ en 6 ou 5) ; 3,34 (DD, en partie masqué, 1H du $-CH_2-$ en 3) ; 3,5 (Mt, 1H, -CH- en 3a) ; 3,82 (DD, 1H, 1H du $-CH_2-$ en 3) ; 3,95 (Mt, 1H, -CH- en 7a) ; 7,15 à 7,65 (Mt, 10H, aromatiques) ; 9,43 (Mf, 2H, $-NH_2^+$).

Spectre infra-rouge (KBr) bandes caractéristiques en $cm^{-1}$:

3600-3300, 3100-3000, 3000-2850, 3100-2400, 1715, 1595, 1580, 1495, 1470, 1445, 775, 750, 705.

La benzyl-2 diphényl-7,7 perhydroisoindolone-4-(3aRS,7aRS) peut être préparée de la manière suivante :

A une solution de 155 g de diphényl-4,4 cyclohexène-2 one-1 et de 202 $cm^3$ de N-butoxyméthyl N-triméthylsilylméthyl benzylamine dans 1000 $cm^3$ de dichlorométhane sec, on ajoute 5 gouttes d'acide trifluoroacétique et chauffe le mélange réactionnel au reflux pendant 45 minutes. On ajoute 50 $cm^3$ de N-butoxyméthyl N-triméthylsilylméthyl benzylamine et 3 gouttes d'acide trifluoroacétique et agite encore 45 minutes au reflux avant d'ajouter de nouveau 25 $cm^3$ de N-butoxyméthyl N-triméthylsilylméthyl benzylamine et 3 gouttes d'acide trifluoroacétique. Le mélange réactionnel est agité au reflux 45 minutes puis traité par 50 g de carbonate de potassium, filtré et concentré à sec sous pression réduite (2,7 kPa). Le résidu est dissous dans 200 $cm^3$ d'oxyde d'isopropyle et la solution refroidie à 0°C pendant 1 heure. Les cristaux sont essorés, lavés 2 fois par 15 $cm^3$ d'oxyde d'isopropyle et séchés pour donner 193 g de benzyl-2 diphényl-7,7 perhydroisoindolone-4-(3aRS,7aRS) sous la forme de cristaux blancs fondant à 132°C.

La N-butoxyméthyl N-triméthylsilyméthyl benzylamine peut être préparée selon la méthode de Y. Terao et coll., Chem. Pharm. Bull., 33, 2762 (1985).

Exemple 2

A une solution refroidie à +4°C de 17,8 g de diphényl-7,7 tert-butyloxycarbonyl-2 perhydroisoindolone-4-(3aR,7aR) dans un litre de méthanol on ajoute goutte à goutte pendant 40 minutes une solution de 1 g de borohydrure de sodium dans 200 $cm^3$ de méthanol puis 10 gouttes de lessive de soude. Le mélange réactionnel est agité 3 heures à +4°C, puis on ajoute 2 $cm^3$ d'une solution aqueuse d'acide chlorhydrique 0,1 N et on concentre à sec sous pression réduite (2,7 kPa). Le résidu est dissous dans 350 $cm^3$ de dichlorométhane, lavé par 100 $cm^3$ d'eau, puis par 50 $cm^3$ de solution saturée de chlorure de sodium, séché sur sulfate de magnésium, concentré à sec sous pression réduite (2,7 kPa). Le résidu est cristallisé dans 40 $cm^3$ d'éther éthylique. Les cristaux obtenus sont essorés et séchés. On obtient 8,4 g de diphényl-7,7 tert-butyloxycarbonyl-2 perhydroisoindolol -4-(3aR,4S,7aR) sous la forme de cristaux blancs fondant à 190°C. Les eaux mères de cristallisation sont concentrées à sec sous pression réduite (2,7 kPa). Le résidu est chromatographié sur colonne de gel de silice (granulométrie 0,04-0,06 mm, diamètre 4 cm, hauteur 33 cm), en éluant sous une pression de 0,4 bar d'azote par un mélange de dichlorométhane et de méthanol (96/4 en volumes) et en recueillant des fractions de 20 $cm^3$. Les fractions 18 à 21 sont réunies et concentrées à sec sous pression réduite (2,7 kPa). On obtient 1,88 g de diphényl-7,7 tert-butyloxycarbonyl-2 perhydroisoindolol -4-(3aR,4R,7aR) sous forme de meringue blanche. Les fractions 26 à 31 sont réunies et concentrées à sec sous pression réduite (2,7 kPa). Le résidu est cristallisé dans 5 $cm^3$ d'éther éthylique. On obtient 2,88 g supplémentaires de diphényl-7,7 tert-butyloxycarbonyl-2 perhydroisoindolol-4-(3aR,4S,7aR) sous forme de cristaux blancs fondant à 190°C.

Le diphényl-7,7 tert-butyloxycarbonyl-2 perhydroisoindolone-4-(3aR,7aR) peut être obtenu de la manière suivante :

A une solution de 20 g de chlorhydrate de diphényl-7,7 perhydroisoindolone-4-(3aR,7aR) dans 100 $cm^3$ de dichlorométhane sec et 6,17 $cm^3$ de triéthylamine on ajoute successivement 0,74 g de diméthylamino-4 pyridine, puis 14,7 g de di-tert-butyl dicarbonate. Le mélange réactionnel est agité pendant 24 heures à température ambiante, puis lavé par une solution aqueuse d'acide citrique, puis par une solution aqueuse d'hydrogénocarbonate de sodium, séché sur sulfate de magnésium, filtré et concentré à sec sous pression réduite (2,7 kPa). Le résidu est cristallisé dans 90 $cm^3$ d'éther éthylique. Les cristaux sont essorés, lavés par 10 $cm^3$ d'éther éthylique puis séchés. On obtient 14,1 g de diphényl-7,7 tert-butyloxycarbonyl-2 perhydroisoindolone-4-(3aR,7aR), sous la forme de cristaux blancs fondant à 119°C.

A une solution de 2 g de diphényl-7,7 tert-butyloxycarbonyl-2 perhydroisoindolol-4-(3aR,4S,7aR) dans 20 $cm^3$ de dioxanne on ajoute une solution de 40 $cm^3$ de dioxanne chlorhydrique 6,3 N et on agite à température ambiante pendant 5 heures. Le mélange réactionnel est concentré à sec sous pression réduite (2,7 kPa), trituré dans l'acétonitrile, filtré et séché. On obtient 1,57 g de chlorhydrate de diphényl-7,7 perhydroisoindolol-4-(3aR,4S,7aR) sous forme de cristaux blancs fondant à 266°C.

Exemple 3

Le chlorhydrate de diphényl-7,7 perhydroisoindolol-4-(3aR,4R,7aR) peut être préparé par hydrogénation d'une suspension de 0,70 g de benzyl-2 diphényl-7,7 perhydroisoindolol-4-(3aR,4R,7aR) dans 30 $cm^3$ de méthanol et 2,0 $cm^3$ d'acide chlorhydrique 1N à pression atmosphérique pendant 20 heures à 20°C en présence de 0,12 g d'hydroxyde de palladium à 20% sur noir de carbone. Le mélange réactionnel est filtré, concentré à sec sous pression réduite (2,7 kPa), l'huile obtenue est concrètée avec de l'éther éthylique. La suspension est filtrée, le solide essoré et séchée sous pression réduite (2,7 kPa). On obtient 0,52 g de chlorhydrate de diphényl-7,7 perhydroisoindolol -4-(3aRS,4R,7aR), sous la forme d'un solide blanc fondant à 220°C (avec décomposition).

Spectre infra-rouge (bandes caractéristiques, $cm^{-1}$): 3400, 3090, 3050, 3025, 3000-2800, 1600, 1580, 1495, 1465, 985, 750, 700.

Spectre RMN du proton (DMSO-$d_6$, signaux principaux): 1, 06 (t large, J-14, 1H, H en 5); 1,66 (d large, J=14, 1H, H en 5); 2,17 (d large, J=14, 1H, $CH_2$ en 6 ) ; 3,8 (s large, 1H, H en 4 ) ; 5,3 (mf, 1H, OH); 7,05 à 7,45 (mt, 10H, aromatiques); 8,4 et 9,43 (mf, 2H, $NH_2^+$).

Le benzyl-2 diphényl-7,7 perhydroisoindolol-4-(3aR,4R,7aR) peut être préparé de la façon suivante:

A une solution refroidie à 0°C de 1,3 g de benzyl-2 diphényl-7,7 perhydroisoindolone-4-(3aR,7aR) dans 6,0 $cm^3$ de tétrahydrofuranne, on ajoute en 5 minutes 4,0 $cm^3$ d'une solution 1M de tri-sec-butyl borohydrure de lithium dans le tetrahydrofuranne. Le mélange réactionnel après agitation 3 heures à 0°C est à nouveau additionné de 0,5 $cm^3$ de la solution 1M de borohydrure. Après 1 heure à 0°C, puis ajout de 50 $cm^3$ d'eau et 50 $cm^3$ d'acétate d'éthyle, la phase organique est décantée, lavée par 20 $cm^3$ d'eau, séchée sur sulfate de magnésium et concentrée à sec sous pression réduite (2,7 kPa). L'huile obtenue est cristallisée dans 30 $cm^3$ d'oxyde de diisopropyle, les cristaux sont essorés, séchés sous pression réduite (2,7 kPa). On obtient 0,70 g de benzyl-2 diphényl-7,7 perhydroisoindolol -4-(3aR,4R,7aR), sous la forme de cristaux blancs fondant à 154°C.

Le benzyl-2 diphényl-7,7 perhydroisoindolone-4-(3aR,7aR) peut être préparé de la façon suivante:

A une solution refroidie à 0°C de 21,7 g de chlorhydrate de diphényl -7,7 perhydroisoindolone-4-(3aR,7aR), dans 300 $cm^3$ de di-chlorométhane, et 18,5 $cm^3$ de triéthylamine, on ajoute 7,9 $cm^3$ de bromure de benzyle. Le mélange réactionnel après agitation 1 heure à 0°C et 2 heures à 20°C est lavé par 50 $cm^3$ d'eau, séché sur sulfate de magnésium concentré à sec sous pression réduite (2,7 kPa). Le résidu est chromatographié sur une colonne de gel de silice (0,04-0,06 mm,diamètre 5 cm, hauteur 40 cm) en éluant sous une pression de 0,6 bar d'azote, par un mélange d'acétate d'éthyle et de cyclohexane (75/25 en volumes) et en recueillant des fractions de 250 $cm^3$. Les fractions 3 à 6 sont réunies et concentrées à sec sous pression réduite (2,7 kPa).

On obtient 22,1 g de benzyl-2 diphényl-7,7 perhydroisoindolone-4-(3aR,7aR), sous la forme d'un solide blanc fondant à 124°C. $[\alpha]^{20}D$ = -279°.

Exemple 4

A une solution refroidie à +5°C de 1,0 g de tertiobutyl-oxycarbonyl-2 diphényl-7,7 perhydroisoindolol-4-(3aR,4R,7aR) dans 20 $cm^3$ de dichlorométhane sec, on ajoute une solution de 0,37 $cm^3$ de trifluorothio-4 morpholine dans 10 $cm^3$ de dichlorométhane sec. Après 2 heures d'agitation à +5°C, le mélange réactionnel est lavé par 20 $cm^3$ de solution aqueuse de bicarbonate de sodium à 5% puis séché sur sulfate de magnésium et concentré à sec sous pression réduite (2,7 kPa). Le résidu est chromatographié sur une colonne de gel de silice (granulométrie 0,04 mm - 0,06 mm, diamètre 2,4 cm, hauteur 35 cm), en éluant sous une pression d'azote de 0,8 bar, par un mélange de cyclohexane et d'acétate d'éthyle (90/10 en volumes) et en recueillant des fractions de 25 $cm^3$. Les fractions 25 à 34 sont réunies et concentrées à sec. On obtient 0,27 g de diphényl-4,4 fluoro-7 tertiobutyloxycarbonyl-2 perhydroisoindole-(3aR,7S,7aR) sous la forme d'une meringue blanche.

Spectre infra-rouge (KBr, bandes caractéristiques, $cm^{-1}$): 3090, 3060, 3030, 2975, 2930, 2875, 1695, 1595, 1580, 1495, 1450, 1405, 1365, 1175, 755, 730, 700.

En opérant comme à l'exemple 8 ci-après à partir de 0,5 g de diphényl -4,4 fluoro-7 tertiobutyloxycarbonyl-2 perhydroisoindole-(3aR,7S,7aR), on obtient 0,35 g de chlorhydrate de diphényl-4,4 fluoro-7 perhydroisoindole-(3aR,7S,7aR) sous la forme d'un solide gris.

Spectre infra-rouge (KBr, bandes caractéristiques, $cm^{-1}$): 3420, 3090, 3050, 3025, 2970, 2920, 2800-2250, 1590, 1580, 1495, 1460, 1445, 1060, 750, 730, 700.

Exemple 5

A une solution refroidie à +5°C de 9,4 g de diphényl-7,7 tert-butyloxycarbonyl-2 perhydroisoindolol-4-(3aR,4S,7aR) dans 250 $cm^3$ de dichlorométhane sec on ajoute une solution de 3,5 $cm^3$ de trifluorure de morpholinosoufre dans 50 $cm^3$ de dichlorométhane. Le mélange réactionnel est agité 4 heures à +5°C puis dilué avec 300 $cm^3$ de dichlorométhane, lavé par 250 $cm^3$ de solution aqueuse d'hydrogénocarbonate de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 42 cm), en éluant sous une pression de 0,5 bar d'azote par un mélange de cyclohexane et d'acétate d'éthyle (90/10 en volumes ) et en recueillant des fractions de 120 $cm^3$. Les fractions 13 à 17 sont réunies et concentrées à sec sous pression réduite (2,7 kPa). Le résidu est cristallisé dans du cyclohexane. On obtient 2,55 g de diphényl-4,4 fluoro-7 tert-butyloxycarbonyl-2 perhydroisoindole-(3aR,7R,7aR) sous forme de cristaux blancs fondant à 202°C.

A une solution de 3,7 g de diphényl-4,4 fluoro-7 tert-butyloxycarbonyl-2 perhydroisoindole-(3aR,7R,7aR) dans 40 $cm^3$ de dioxanne on ajoute une solution de 40 $cm^3$ de dioxanne chlorhydrique 6,3 N et on agite à température ambiante pendant 2 heures. Le mélange réactionnel est concentré à sec sous pression réduite (2,7 kPa), trituré dans de l'oxyde de diisopropyle, filtré et séché. On obtient 3,1 g de chlorhydrate de diphényl-4,4 fluoro-7 perhydroisoindole-(3aR,7R,7aR) sous forme de cristaux blancs fondant à 200°C avec décomposition .

Exemple 6

A une solution refroidie à +4°C de 1 g de diphényl-7,7 tert-butyloxycarbonyl-2 perhydroisoindolol-4-(3aR,4S,7aR) dans 60 $cm^3$ de chloroforme, on ajoute successivement 1,3 g de carbonate de calcium, puis 2 g de pentachlorure de phosphore et on agite à température ambiante pendant 20 heures. Le mélange réactionnel est ensuite Filtré, dilué par 80 $cm^3$ de chloroforme, lavé deux fois par 80 $cm^3$ d'eau, séchée sur sulfate de magnésium et concentré à sec sous pression réduite (2,7 kPa). Le résidu est chromatographié sur colonne de gel de silice (granulométrie 0,04-0,06 mm, diamètre 2,5 cm, hauteur 34 cm), en éluant sous une pression de 0,4 bar d'azote par un mélange de cyclohexane et d'acétate d'éthyle (30/70 en volumes) et en recueillant des fractions de 20 $cm^3$. Les fractions 7 à 10 sont réunies et concentrées à sec sous pression réduite (2,7 kPa). On obtient 0,44 g de chloro-7 chlorocarbonyl-2 diphényl-4,4 perhydroisoindole -(3aR,7R,7aR) sous la forme d'un solide blanc. Spectre infra-rouge (Solution $CCl_4$, bandes caractéristiques, $cm^{-1}$): 3090, 3065, 3035, 2930, 2855, 1745, 1600, 1585, 1495, 1450, 700.

Une solution de 0,4 g de chloro-7 chlorocarbonyl-2 diphényl-4,4 perhydroisoindole-(3aR,7R,7aR) dans 6 $cm^3$ d'une solution aqueuse 1N d'acide chlorhydrique et 14 $cm^3$ de tétrahydrofuranne est portée à 80°C sous agitation pendant 9 heures. Le mélange réactionnel est concentré à sec sous pression réduite (2,7 kPa). On obtient 0,35 g de chlorhydrate de chloro-7 diphényl-4,4 perhydroisoindole-(3aR,7R,7aR) sous forme de solide

blanc.

Spectre infra-rouge (KBr, bandes caractéristiques, $cm^{-1}$): 3055, 3025, 3000, 2250,1600, 1495, 1580, 1460, 1445, 1435, 760, 750, 735, 700.

Exemple 7

Une solution de 1 g de tert-butyloxycarbonyl-2 diphényl-7,7 perhydroisoindolol-4-(3aR,4S,7aR) dans 10 $cm^3$ de chlorure de thionyle est agitée 3 heures à 80°C. Le mélange réactionnel est ensuite concentré à sec sous pression réduite (2,7 kPa). On obtient 1,03 g de tert-butyloxycarbonyl-2 chloro-7 diphényl-4,4 perhydroisoindole-(3aR,7S,7aR) sous la forme d'un solide, utilisé brut dans l'essai suivant.

A une solution de 1,03 g de tert-butyloxycarbonyl-2 chloro-7 diphényl-4,4 perhydroisoindole-(3aR,7S,7aR) dans 5 $cm^3$ de dioxane on ajoute 10 $cm^3$ d'une solution 6,3 N d'acide chlorhydrique dans le dioxanne. Le mélange réactionnel est agité-. à température ambiante pendant 2 heures, puis concentré à sec sous pression réduite (2,7 kPa). On obtient 0,84 g de chlorhydrate de chloro-7 diphényl-4,4 perhydroisoindole-(3aR,7S,7aR) sous forme d'un solide, utilisé brut dans l'essai suivant.

Exemple 8

A une solution de 3,4 $cm^3$ de trifluorure de diéthylamino soufre dans 20 $cm^3$ de dichlorométhane sec, on ajoute une solution de 5,0 g de tertiobutyloxycarbonyl-2 diphényl-7,7 perhydroisoindolone-4-(3aRS,7aRS) dans 30 $cm^3$ de dichlorométhane sec. Après 5 heures d'agitation au reflux et 20 heures à 20°C, le mélange réactionnel est lavé par 50 $cm^3$ de solution aqueuse saturée de bicarbonate de sodium et par 50 $cm^3$ d'eau, puis séché sur sulfate de magnésium, et concentré à sec. Le résidu est chromatographié sur une colonne de gel de silice (granulométrie 0,04 - 0,06 mm, diamètre 2,8 cm, hauteur 35 cm), en éluant sous une pression d'azote de 0,8 bar par un mélange de cyclohexane et d'acétate d'éthyle (95/5 puis 90/10 en volumes) et en recueillant des fractions de 25 $cm^3$. Les fractions 24 à 52 sont réunies et concentrées à sec sous pression réduite (2,7 kPa). Le résidu est cristallisé dans de l'acétate d'éthyle et de l'oxyde de diisopropyle, les cristaux sont essorés puis séchés. On obtient 1,80 g de tertiobutyloxycarbonyl-2 diphényl-4,4 difluoro-7,7 perhydroisoindole-(3aRS,7aRS) sous la forme de cristaux blancs fondant à 162°C.

A 1,8 g de tertiobutyloxycarbonyl-2 diphényl-4,4 difluoro-7,7 perhydroisoindole-(3aRS,7aRS) on ajoute 20 $cm^3$ de dioxanne et 20 $cm^3$ d'acide chlorhydrique 6,3N. Après 20 heures d'agitation à température ambiante, la suspension blanche obtenue est concentrée à sec à 40°C sous pression réduite (2,7 kPa). Le résidu est lavé avec de l'oxyde de diisopropyle, le solide obtenu essoré, puis séché. On obtient 1,51 g de chlorhydrate de diphényl-4,4 difluoro-7,7 perhydroisoindole -(3aRS,7aRS) sous la forme d'un solide blanc.

Spectre infra-rouge (KBr, bandes caractéristiques, $cm^{-1}$): 3090, 3050, 3025, 2965, 2935, 2900, 2800-2250, 1595, 1580, 1495, 1465, 1445, 760, 730, 700.

Spectre RMN du proton (DMSO-$d_6$ + $CF_3COOD$): 1,2-1,55 et 2,12 (2mt, 2x1H, $CH_2$ en 6); 3-3,3 (mt, 1H, H en 7a); 3,58 (mt, 2H, $CH_2$ en 1); 3,76 (mt, 1H, H en 3a); 7,1 à 7,5 (mt, 10H, aromatiques).

Exemple 9

Une solution de 4,87 g de t-butoxycarbonyl-2 diphényl-4,4 trifluorométhylsulfonyloxy-7 perhydroisoindole-(3aR,7S,7aR) dans 150 $cm^3$ de dichlorométhane sec est traitée par 22,6 $cm^3$ de solution 1M de fluorure de tétrabutylammonium dans le tétrahydrofuranne puis agitée 17 heures à 20°C et concentré à sec sous pression réduite (2,7 kPa). Le résidu est chromatographié sur une colonne de gel de silice (granulométrie 0,2-0,063 mm, diamètre 4,5 cm, hauteur 35 cm) en éluant sous une pression de 0,4 bar d'azote par un mélange de cyclohexane et d'acétate d'éthyle (75/25) et en recueillant des fractions de 20 $cm^3$. Les fractions 28 à 38 sont réunies et concentrées à sec sous pression réduite (2,7 kPa) pour donner 1,44 g de t-butoxycarbonyl-2 diphényl-4,4 fluoro-7 perhydroisoindole-(3aR,7R,7aR) sous la forme de cristaux blancs fondant à 200°C, $[\alpha]_D^{20}$= - 225° (c=1, $CHCl_3$).

Une solution de 2,25 g de t-butoxycarbonyl-2 diphényl-4,4 fluoro-7 perhydroisoindole-(3aR,7R,7aR) dans 25 $cm^3$ de dioxanne est traitée par une solution 5,8 N d'acide chlorhydrique dans le dioxanne et agitée 2 heures à 20°C puis concentrée à sec sous pression réduite (2,7 kPa). Le résidu est concrété par addition de 100 $cm^3$ d'oxyde d'isopropyle, le solide est filtré et séché pour donner 1,8 g de chlorhydrate de diphényl-4,4 fluoro-7 perhydroisoindole-(3aR,7R,7aR) sous la forme d'une poudre crème.

Spectre RMN du proton (DMSO-$d_6$): 1,0-1,35 (mt, 1H du $CH_2$en 6) ; 4,9 (d large, J=50, 1H, CHF) ; 7,1 à 7,5 (mt, 14H, aromatiques) ; 9,05 et 9,9 (2 mf, 2x1H, $NH_2^+$).

Le t-butoxycarbonyl-2 diphényl-4,4 trifluorométhylsulfonyloxy-7 perhydroisoindole-(3aR,7S,7aR) peut être

obtenu de la manière suivante :

A une solution de 6,7 g de t-butoxycarbonyl-2 diphényl-7,7 perhydroisoindolol-4-(3aR,4S,7aR) dans 100 $cm^3$ de dichlorométhane sec refroidie à -30°C on ajoute 1,5 $cm^3$ de pyridine puis, en 10 minutes, une solution de 3,2 g d'anhydride trifluorométhanesulfonigue dans 25 $cm^3$ de dichlorométhane sec. Le mélange réactionnel est agité 2 heures à -30°C puis dilué par 250 $cm^3$ d'eau et 100 $cm^3$ de dichlorométhane. La phase organique est lavée par 200 $cm^3$ de solution saturée de bicarbonate de sodium et par 200 $cm^3$ de solution saturée de chlorure de sodium puis séchée et concentrée à sec sous pression réduite (2,7 kPa) pour donner 8,6 g de t-butoxycarbonyl-2 diphényl-4,4 trifluorométhylsulfonyloxy-7 perhydroisoindole-(3aR,7S,7aR) sous la forme d'une meringue jaune utilisée telle quelle dans la suite de la synthèse.

Le t-butoxycarbonyl-2 diphényl-7,7 perhydroisoindolol-4-(3aR,4S,7aR) peut être obtenu de la manière suivante :

A une solution de 13 g de diphényl-7,7 perhydroisoindolol-4-(3aR,4S,7aR) et 0,5 g de diméthylamino-4 pyridine dans 450 $cm^3$ de dichlorométhane on ajoute 10,55 g de diterbutyldicarbonate. Après agitation durant 2 heures à 25°C, le mélange réactionnel est concentré à sec sous pression réduite (2,7 kPa) et le résidu est cristallisé dans 50 $cm^3$ d'éther éthylique. On obtient 9 g de t-butoxycarbonyl-2 diphényl-7,7 perhydroisoindolol-4-(3aR,4S,7aR) sous la forme de cristaux blancs fondant à 190°C.

Les produits selon l'invention peuvent être mis en oeuvre pour la préparation des dérivés de l'isoindole de formule générale (X) comme dans les exemples d'utilisation ci-après.

Exemple d'utilisation 1

A une solution de 0,72 g de chlorhydrate de diphényl-4,4 fluoro-7 perhydroisoindole-(3aR,7R,7aR), 0,5 g d'acide (diméthylamino-3 propoxy)-2 phénylacétique, 0,03 g d'hydroxy-1 benzotriazole dans 75 $cm^3$ de dichlorométhane refroidie à + 4°C on ajoute en 10 minutes une solution de 0,5 g de (diméthylamino-3 propyl)-1 éthyl-3 carbodiimide dans 50 $cm^3$ de dichlorométhane sec puis 0,37 $cm^3$ de diisopropyléthylamine. Le mélange réactionnel est agité 3 heures à 0°C puis lavé 2 fois par 50 $cm^3$ d'eau et 2 fois par 50 $cm^3$ de solution saturée de chlorure de sodium. La phase organique est séchée sur sulfate de magnésium et concentrée à sec sous pression réduite (2,7 kPa). Le résidu est repris par 21 $cm^3$ d'acide chlorhydrique 0,1 N, 50 $cm^3$ d'éther diéthylique et 30 $cm^3$ d'eau. La phase aqueuse est séparée et lyophilisée pour donner 0,85 g de chlorhydrate de [(diméthylamino-3 propoxy)-2 phényl]acétyl-2 diphényl-4,4 fluoro-7 perhydroisoindole-(3aR,7R,7aR), sous la forme d'un lyophilisat blanc.

Spectre infra-rouge (KBr, bandes caractéristiques, $cm^{-1}$): 3060, 3030, 2960, 2890, 2800, 2200, 1635, 1605, 1495, 1460, 1445, 1250, 755, 705.

Spectre RMN du proton (DMSO-$d_6$) (à température ambiante, on observe le mélange des deux rotamères):

0,95-1,35 et 1,8-2,1 (2mt, 2x1H, $CH_2$ en 6) ; 2,6-2,8 (mt, 6H, $N(CH_3)_2$) ; 3,9 et 4,05 (2mt, 2x1H, $OCH_2$) ; 4,8 et 4,85 (2d larges, J=50, 1H, CHF) ; 6,8 à 7,5 (mt, 14H, aromatiques).

Exemple d'utilisation 2

A une solution refroidie à +5°C de 1 g de chlorhydrate de diphényl -4,4 fluoro-7 perhydroisoindole-(3aR,7R,7aR) et de 0,924 g d'acide {[(pyrrolidinyl-1)-3 propoxy-2]phényl}-2 acétique dans 40 $cm^3$ de dichlorométhane sec, on ajoute 0,04 g d'hydrate d'hydroxybenzotriazole, puis 0,79 g chlorhydrate de (diméthylamino-3 propyl)-1 éthyl-3 carbodiimide et 0,51 $cm^3$ de diisopropyléthylamine. Après 2,5 heures d'agitation à +5°C et 20 heures à 20°C, le mélange réactionnel est lavé 2 fois par 50 $cm^3$ d'eau, séché sur sulfate de magnésium, puis concentré à sec à 40°C sous pression réduite (2,7 kPa). Le résidu est chromatographié sur colonne de gel de silice (granulométrie 0,04-0,06 mm, diamètre 2,4 cm, hauteur 31 cm), en éluant sous une pression de 0,5 bar d'azote par un mélange d'acétate d'éthyle, d'acide acétique et d'eau (60/10/10 en volumes) et en recueillant des fractions de 25 $cm^3$. Les fractions 11 à 31 sont réunies et concentrées à sec sous pression réduite (2,7 kPa). Le résidu est repris avec 20 $cm^3$ de dichlorométhane, la solution est lavée par 20 $cm^3$ de solution aqueuse d'hydroxyde de sodium 1N, puis est séchée sur sulfate de magnésium, et concentrée à sec. Ce lavage basique est effectué une nouvelle fois. On obtient 0,68 g de {{[(pyrrolidinyl-1)-3 propoxy-2]phényl}acétyl}-2 diphényl-4,4 fluoro-7 perhydro-isoindole-(3aR,7R,7aR) sous la forme d'un solide blanc.

Spectre Infra-rouge (KBr, bandes caractéristiques, $cm^1$): 3085, 3055, 3035, 2950, 2875, 2785, 1640, 1600, 1495, 1455, 1440, 1245, 750, 700.

Spectre RMN du proton (DMSO-$d_6$ + $CF_3COOD$): 1,1-1,45 (mt, 1H, 1H en 6); 1,9 (mt, 4H, $2CH_2$ en 3 et 4 du pyrrolidino); 2,27 (mt, 1H, 1H en 5); 3,77 (d, J=10, 1H, H en 1); 4,03 (mt, 2H, $OCH_2$); 4,78 (d large, J=50, 1H, CHF); 7,1 à 7,5 (mt, 14H, aromatiques).

Exemple d'utilisation 3

En opérant comme à l'exemple 9 ci-après, à partir de 0,16 g d'acide diméthylamino-2 phénylacétique et de 0,30 g de chlorhydrate de diphényl -4,4 fluoro-7 perhydroisoindole- (3aR,7S,7aR) on obtient 0,11 g de [(diméthylamino-2 phényl)acétyl]-2 diphényl-4,4 fluoro-7 perhydroisoindole- (3aR,7S,7aR) sous la forme d'une meringue blanche.

Spectre infra-rouge (KBr, bandes caractéristiques, $cm^{-1}$): 3090, 3060, 3030, 2940, 2875, 2825, 2770, 1645, 1595, 1580, 1495, 1450, 1420, 755, 730, 700.

Spectre RMN du proton (à température ambiante, on observe le mélange de deux rotamères) : 2,35 et 2,58 (2s, 6H, $N(CH_3)_2$), 4,2-4,6 (mt, 1H, CHF), 6,9-7,5 (mt, 14H, aromatiques).

Exemple d'utilisation 4

A une solution de 0,57 g de bromhydrate d'acide (pyrrolidino-2) phénylacétique dans 20 $cm^3$ de dichlorométhane sec, refroidie à 4°C, on ajoute 0,28 $cm^3$ de triéthylamine et 0,32 g de carbonyldiimidazole. On agite une heure à + 4°C, puis on ajoute une solution de 0,67 g de chlorhydrate de diphényl-4,4 fluoro-7 perhydroisoindole-(3aR,7R,7aR) dans 20 $cm^3$ de dichlorométhane sec et 0,28 $cm^3$ de triéthylamine. Le mélange réactionnel est agité à température ambiante pendant 24 heures, puis dilué par 100 $cm^3$ de dichlorométhane, lavé deux fois par 50 $cm^3$ d'eau, séché sur sulfate de magnésium, filtré et concentré à sec sous pression réduite (2,7 kPa). Le résidu est chromatographié sur colonne de gel de silice (granulométrie 0,04-0,06 mm, diamètre 3,5 cm, hauteur 38 cm), en éluant sous une pression de 0,5 bar d'azote par un mélange de cyclohexane et d'acétate d'éthyle (70/30 en volumes) et en recueillant des fractions de 20 $cm^3$. Les fractions 26 à 54 sont réunies et concentrées à sec sous pression réduite (2,7 kPa). Le résidu est cristallisé dans un mélange d'acétonitrile et d'oxyde de diisopropyle (25/75 en volumes). On obtient 0,16 g de diphényl-4,4 fluoro-7 [(pyrrolidino-2 phényl)-acétyl]-2 perhydroisoindole -(3aR,7R,7aR) sous forme de cristaux blancs fondant à 170°C.

Exemple d'utilisation 5

A une solution de 0,19 g d'acide (diméthylamino-2 phényl)-acétique dans 15 $cm^3$ de dichlorométhane sec, refroidie à + 4°C, on ajoute 0,17 g de carbonyldiimidazole. On agite une heure à + 4°C, puis on ajoute une solution de 0,35 g de chlorhydrate de chloro-7 diphényl-4,4 perhydroisoindole-(3aR,7R,7aR) dans 10 $cm^3$ de di-chlorométhane sec, puis une solution de 0, 15 $cm^3$ de triéthylamine dans 10 $cm^3$ de dichlorométhane sec. Le mélange réactionnel est agité à température ambiante pendant 20 heures, puis dilué par 120 $cm^3$ de dichlorométhane, lavé par 80 $cm^3$ d'eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 2 cm, hauteur 22 cm), en éluant sous une pression de 0,4 bar d'azote par un mélange d'acétate d'éthyle et de cyclohexane (75/25 en volumes) et en recueillant des fractions de 20 $cm^3$. Les fractions 6 à 9 sont réunies et concentrées à sec sous pression réduite (2,7 kPa). Le produit, obtenu sous forme de base, est converti en chlorhydrate par dissolution dans 25 $cm^3$ d'éther éthylique, puis ajout de 5 $cm^3$ d'une solution 3,2 N d'acide chlorhydrique dans l'éther éthylique, lavage à l'éther éthylique et séchage. On obtient 0,14 g de chlorhydrate de chloro-7 [(diméthylamino-2 phényl)-acétyl]-2 diphényl-4,4 perhydroisoindole-(3aR,7R,7aR) sous la forme de cristaux blancs fondant à 190°C.

Exemple d'utilisation 6

A une solution de 0,43 g d'acide (diméthylamino-2 phényl) acétique dans 15 $cm^3$ de dichlorométhane sec, refroidie à + 4°C, on ajoute 0,39 g de carbonyldiimidazole. On agite une heure à + 4°C, puis on ajoute une solution de 0,84 g de chlorhydrate de chloro-7 diphényl-4,4 perhydroisoindole-(3aR,7S,7aR) dans 10 $cm^3$ de dichlorométhane sec, puis une solution de 0,34 $cm^3$ de triéthylamine dans 10 $cm^3$ de dichlorométhane sec. Le mélange réactionnel est agité à température ambiante pendant 20 heures, puis dilué par 100 $cm^3$ de dichlorométhane, lavé par 50 $cm^3$ d'eau, puis par une solution aqueuse saturée en chlorure de sodium, séché sur sulfate de magnésium, filtré et concentré à sec sous pression réduite (2,7 kPa). Le résidu est chromatographié sur colonne de gel de silice (granulométrie 0,04-0,06 mm, diamètre 3 cm, hauteur 23 cm), en éluant sous une pression de 0,4 bar d'azote par un mélange d'acétate d'éthyle et de cyclohexane (25/75 en volumes) et en recueillant des fractions de 80 $cm^3$. La fraction 2 est concentrée à sec sous pression réduite (2,7 kPa). Le produit obtenu sous forme de base, est converti en chlorhydrate par dissolution dans 4 $cm^3$ d'acétonitrile, puis ajout de 6 $cm^3$ d'une solution 3,2 N d'acide chlorhydrique dans l'éther éthylique, lavage à l'éther isopropylique et séchage. On obtient 0,08 g de chlorhydrate de chloro-7 [(diméthylamino-2 phényl)-acétyl]-2 diphényl-4,4 per-

hydroisoindole-(3aR,7S,7aR) sous la forme d'un solide beige.

Spectre infra-rouge (KBr, bandes caractéristiquues, $cm^{-1}$): 3055, 3025, 2950, 1635, 1490, 1460, 1440, 760, 750, 700.

Spectre RMN du proton (DMSO-$d_6$) (à 403°K on observe le mélange des deux rotamères, DMSO-$d_6$ + $CF_3COOD$, signaux principaux): 3 et 3, 13 (2s, 6H, $N(CH_3)_2$) ; 4,54 et 4,63 (2mt, 1H, CHCl) ; 7 à 7,8 (mt, 14H, aromatiques ).

Exemple d'utilisation 7

A une solution de 0,36 g d'acide (méthoxy-2 phényl)-2 propionique-(S) dans 20 $cm^3$ de dichlorométhane sec, refroidie à +4°C, on ajoute 0,32 g de carbonyldiimidazole. On agite une heure à +4°C, puis on ajoute une solution de 0,67 g de chlorhydrate de diphényl-4,4 fluoro-7 perhydroisoindole-(3aR,7R,7aR) dans 20 $cm^3$ de dichlorométhane sec et 0,28 $cm^3$ de triéthylamine. Le mélange réactionnel est agité à température ambiante pendant 20 heures, dilué dans 200 $cm^3$ de dichlorométhane puis lavé par 50 $cm^3$ d'eau, séché sur sulfate de magnésium, filtré et concentré à sec sous pression réduite (2,7 kPa). Le résidu est chromatographié sur colonne de gel de silice (granulométrie 0,04-0,06 mm diamètre 3 cm, hauteur 20 cm), en éluant sous une pression de 0,4 bar d'azote par un mélange d'acétate d'éthyle et de cyclohexane (60/40 en volumes) et en recueillant des fractions de 20 $cm^3$. Les fractions 10 à 15 sont réunies et concentrées à sec sous pression réduite (2,7 kPa). Le résidu est cristallisé dans 0,6 $cm^3$ d'oxyde d'isopropyle. Les cristaux obtenus sont essorés, lavés par de l'oxyde d'isopropyle, puis séchés. On obtient 0,19 g de diphényl-4,4 fluoro-7 [(méthoxy-2 phényl)-2 propionyl-(S)]-2 perhydroisoindole-(3aR,7R,7aR) sous forme de cristaux blancs fondant à 195°C.

L'acide (méthoxy-2 phényl)-2 propionique-(S) peut être obtenu de la manière suivante :

L'acide (méthoxy-2 phényl)-2 propionique-(S) peut être préparé, par analogie avec les méthodes décrites par D.A Evans et coll., Tetrahedron, 44, 5525,(1988), selon le mode opératoire suivant:

A une solution refroidie à +5°C de 4,1 g de méthyl-4 phényl-5 [(méthoxy-2 phényl)-2-(S)-propionyl]-3 oxazolidinone-2-(4S,5S) dans 60 $cm^3$ de tétrahydrofuranne et 30 $cm^3$ d'eau, on ajoute 1,52 g d'hydroxyde de lithium. Le mélange réactionnel est agité 3 heures à cette température, puis, après retour à température ambiante, on rajoute de l'acétate d'éthyle, on décante, la phase aqueuse est acidifiée par une solution aqueuse d'acide chlorhydrique 1N, extraite par de l'acétate d'éthyle, la phase organique est séchée sur sulfate de magnésium, et concentrée à sec sous pression réduite (2,7 kPa). Le solide obtenu est recristallisé dans l'hexane, essoré et séché. On obtient 0,4 g d'acide (méthoxy-2 phényl)-2 propionique-(S) sous la forme de cristaux blancs fondant à 102°C. $[\alpha]_D^{20}$ = +84,6° (c=1; $CHCl_3$).

La méthyl-4 phényl-5 [(méthoxy-2 phényl)-2-(S)-propionyl]-3 oxazolidinone-2-(4S,5S) peut être obtenue de la manière suivante :

A une solution refroidie à -50°C de 10 g de méthyl-4 phényl-5 [(méthoxy-2 phényl)-acétyl]-3 oxazolidinone-2-(4S,5S) dans 150 $cm^3$ de tétrahydrofuranne on ajoute 19,1 g d'hexaméthyl-1,1,1,3,3,3 disilazanate de sodium, on agite 45 minutes à cette température, puis on ajoute 7,72 $cm^3$ d'iodure de méthyle. Le mélange réactionnel est ensuite agité 15 heures à température ambiante, puis dilué par de l'acétate d'éthyle, lavé par 50 $cm^3$ d'eau, puis par 50 $cm^3$ d'une solution aqueuse saturée de chlorure de sodium, séché sur sulfate de magnésium et concentré à sec sous pression réduite (2,7 kPa). Le résidu obtenu est cristallisé dans l'oxyde d'isopropyle, essoré et séché. On obtient 4,2 g de méthyl-4 phényl-5 [(méthoxy-2 phényl)-2-(S)-propionyl]-3 oxazolidinone-2- (4S,5S) sous la forme d'un solide blanc.

La méthyl-4 phényl-5 (méthoxy-2 phénylacétyl)-3 oxazolidinone-2-(4S,5S) peut être obtenue de la manière suivante :

A une suspension de 1,89 d'hydrure de sodium (dispersion à 80% dans la vaseline) dans 200 $cm^3$ de tétrahydrofuranne sec on ajoute à température ambiante 9,38 g d'acide méthoxy-2 phénylacétique. On refroidit cette suspension à -30°C, on ajouté 7,77 $cm^3$ de chlorure de pivaloyle, puis on ajoute enfin une solution refroidie à -78°C obtenue en additionnant une solution de 35,27 $cm^3$ de butyllithium 1,6 M dans l'hexane à une solution refroidie à -78°C de 10 g de méthyl-4 phényl-5 oxazolidinone-2-(4S,5S) dans 200 $cm^3$ de tétrahydrofuranne sec. Le mélange réactionnel est agité 45 minutes à -30°C, puis après retour à température ambiante, on ajoute 200 $cm^3$ d'une solution aqueuse saturée de chlorure d'ammonium, puis 500 $cm^3$ d'acétate d'éthyle ; aprés décantation la phase organique est lavée deux fois par 100 $cm^3$ d'eau, puis deux fois par 100 $cm^3$ d'une solution aqueuse saturée de chlorure de sodium; séchée sur sulfate de magnésium et concentrée à sec sous pression réduite (2,7 kPa). Le résidu est chromatographié sur colonne de gel de silice (granulométrie 0,04-0,06 mm, diamètre 4,8 cm, hauteur 36 cm), en éluant sous une pression de 0,6 bar d'azote par un mélange de cyclohexane et d'acétate d'éthyle (85/15 puis 80/20 en volumes) et en recueillant des fractions de 50 $cm^3$. Les fractions 14 à 31 sont réunies et concentrées à sec sous pression réduite (2,7 kPa). On obtient 13,6 g de méthyl-4 phényl-5 (méthoxy-2 phénylacétyl)-3 oxazolidinone-2-(4S,5S) sous la forme d'une huile jaune.

Exemple d'utilisation 8

En opérant selon le mode opératoire de l'exemple d'utilisation 9 ci-après à partir de 0,77 g d'acide diméthylamino-2 phénylacétique et de 1,50 g de chlorhydrate de diphényl-4,4 difluoro-7,7 perhydroisoindole -(3aRS,7aRS) on obtient 1,29 g de [(diméthyl-amino-2 phényl)acétyl]-2 diphényl-4,4 difluoro-7,7 perhydro-isoindole -(3aRS,7aRS) sous la forme d'un solide blanc fondant à 189°C.

Exemple d'utilisation 9

A une solution de 0,52 g d'acide diméthylamino-2 phénylacétique dans 20 $cm^3$ de dichlorométhane sec, on ajoute 0,49 g de N,N'-carbonyldiimidazole. On agite 30 minutes à +5°C puis on ajoute une solution de 0,93 g de chlorhydrate de diphényl-7,7 perhydroisoindolol-4-(3aR,4S,7aR) et de 0,84 $cm^3$ de triéthylamine dans 10 $cm^3$ de dichlorométhane. Le mélange réactionnel est agité pendant 2 heures à +5°C puis lavé par 10 $cm^3$ d'eau, séché sur sulfate de magnésium, filtré et concentré à sec sous pression réduite (2,7 kPa). Le résidu obtenu est chromatographié sur une colonne de gel de silice (0,04 mm-0,06 mm, diamètre 2 cm, hauteur 35 cm), en éluant par de l'acétate d'éthyle et en recueillant des fractions de 30 $cm^3$. Les fractions 8 à 27 sont réunies, concentrées à sec sous pression réduite (2,7 kPa). Le résidu est cristallisé dans un mélange de 4 $cm^3$ d'acétonitrile et de 20 $cm^3$ d'éther éthylique. Les cristaux sont essorés, séchés sous pression réduite (2,7 kpa). On obtient 0,70 g de [di(méthylamino-2 phényl)acétyl]-2 diphényl-7,7 perhydroisoindolol-4-(3aR,4S,7aR), sous la forme d'un solide blanc fondant à 160°C, $[\alpha]^{20}D$ = -162° (c - 0,5, méthanol).

Exemple d'utilisation 10

En opérant selon le mode opératoire de l'exemple d'utilisation 9 à partir de 0,26 g d'acide diméthylamino-2 phénylacétique et de 0,50 g de chlorhydrate de diphényl-7,7 perhydroisoindolol-4- (3aR,4R,7aR), on obtient 0,21 g de [(diméthylamino-2 phényl) acétyl]-2 diphényl-7,7 perhydroisoindolol-4-(3aR,4R,7aR), sous la forme d'un solide blanc fondant à 204°C, $[\alpha]^{20}D$ = -212° (c = 0,5, méthanol).

Exemple d'utilisation 11

A une solution de 0,86 g de bromhydrate d'acide (pyrrolidino-2)-phényl-acétique dans 20 $cm^3$ de dichlorométhane sec, refroidie à + 4°C, on ajoute 0,42 $cm^3$ de triéthylamine et 0,49 g de carbonyldiimidazole. On agite une heure à + 4°C, puis on ajoute une solution de 1 g de chlorhydrate de diphényl-7,7 perhydro-isoindolol-4-(3aR,4S,7aR) et 0,42 $cm^3$ de triéthylamine dans 10 $cm^3$ de dichlorométhane sec. Le mélange réactionnel est agité à température ambiante pendant 24 heures, puis puis lavé deux fois par 10 $cm^3$ d'eau, puis par une solution aqueuse d'hydrogénocarbonate de sodium, séché sur sulfate de magnésium, filtré et concentré à sec sous pression réduite (2,7 kPa). Le produit, obtenu sous forme de base, est converti en chlorhydrate par dissolution dans la quantité minimale d'acétone et traitement par une solution d'acide chlorhydrique dans l'éther éthylique et ajout d'éther éthylique. Le solide obtenu est trituré dans l'éther éthylique, puis séché. On obtient 0,2 g de chlorhydrate de diphényl-7,7 [(pyrrolidino-2 phényl) acétyl ]-2 perhydroisoindolol-4-(3aR,4S,7aR) sous forme de solide beige.

Spectre infra-rouge (KBr, bandes caractéristiques, $cm^{-1}$): 3085, 3050, 3025, 2945, 2880, 2750, 2250, 1640, 1600, 1495,1445, 1060, 755, 730, 700.

Spectre RMN du proton (DMSO-$d_6$): 0,92 et 1,72 (2 mt, 2x1H, $CH_2$- en 5) ; 2,17 (mt, 4H, 2 $CH_2$ en 3 et 4 du pyrrolidino) ; 7 à 7,8 (mt, 14H, aromatiques).

Exemple d'utilisation 12

En offrant comme décrit précédemment dans l'exemple 9, à partir de 1,82 g d'acide (méthoxy-2 phényl)acétique et de 3,29 g de chlorhydrate de diphényl-7,7 perhydroisoindolol-4-(3aR,4S,7aR), on obtient 3,9 g de [(méthoxy-2 phényl)acétyl]-2 diphényl-7,7 perhydroisoindolol-4-(3aR,4S,7aR) sous la forme d'un solide blanc, fondant à 246°C. $[\alpha]^{20}D$ = -174° (c = 0,37; méthanol)

Exemple d'utilisation 13

A une solution de 0,41 g d'acide (méthoxy-2 phényl)-2 propionique-(S) dans 15 $cm^3$ de dichlorométhane sec, refroidie à + 4°C, on ajoute 0,37 g de carbonyldiimidazole. On agite une heure à + 4°C, puis on ajoute une solution de 0,75 g de chlorhydrate de diphényl-7,7 perhydroisoindolol-4-(3aR,4S,7aR). Le mélange réactionnel est agité à température ambiante pendant 20 heures, puis puis lavé deux fois par 70 $cm^3$ d'eau, séchée sur sulfate de magnésium, filtré et concentré à sec sous pression réduite (2,7 kPa). Le résidu est chromatographié sur colonne de gel de silice (granulométrie 0,04-0,06 mm diamètre 3,6 cm, hauteur 37 cm), en éluant sous une pression de 0,5 bar d'azote par un mélange d'acétate d'éthyle et de cyclohexane (50/50 en volumes) et en recueillant des fractions de 50 $cm^3$. Les fractions 21 à 41 sont réunies et concentrées à sec sous pression réduite (2,7 kPa). Le résidu est trituré dans de l'oxyde d'isopropyle puis séché. On obtient 0,3 g de diphényl-7,7 [(méthoxy-2 phényl)-2 propionyl-(S)]-2 perhydroisoindolol-4-(3aR,4S,7aR) sous la forme d'une meringue blanche.

Spectre infra-rouge (KBr, bandes caractéristiques, $cm^{-1}$): 3090, 3060, 3030, 2940, 2875, 2840, 1630, 1600, 1495, 1445, 1245, 1060, 755, 730, 700.

Spectre RMN du proton (DMSO-$d_6$) (à température ambiante, on observe le mélange des deux rotamères): 0,9-1,8 (mt, 2H, $CH_2$ en 5); 1,14 et 1,23 (2d, J-7, 3H, $CH_3$); 3,55 et 3,65 (2s, 3H, $CH_3$); 3,85 et 4,23 (2mt, 1H, -CO<u>CH</u>$CH_3$-); 6,8 à 7,5 (mt, 14H, aromatiques).

<u>Exemple d'utilisation 14</u>

A une solution refroidie à + 10°C de 1 g de diphényl-7,7 perhydroisoindolol-4-(3aR,4S,7aR), 0,97 g d'acide (diméthylamino-3 propoxy)-2 phénylacétique, 0,05 g d'hydroxy-1 benzotriazole dans 50 $cm^3$ de dichlorométhane on ajoute 0,766 g de (diméthylamino-3 propyl)-1 éthyl-3 carbodiimide. Le mélange réactionnel est agité 90 minutes 20°C puis lavé 2 fois par 50 $cm^3$ d'eau et par 50 $cm^3$ de solution saturée de chlorure de sodium. La phase organique est séchée sur sulfate de magnésium et concentrée à sec sous pression réduite (2,7 kPa). Le résidu est chromatographié sur une colonne de gel de silice (granulométrie 0,2-0,063 mm, diamètre 2,9 cm, hauteur 23 cm) en éluant sous une pression de 0,7 bar d'azote par des mélanges de dichloro-1,2 éthane et de méthanol (1 litre à 90/10 en volumes et 1,5 litre à 70/30 en volume) et en recueillant des fractions de 25 $cm^3$. Les fractions 10 à 84 sont réunies et concentrées à sec sous pression réduite (2,7 kPa) pour donner 1,1 g de {[(diméthylamino-3 propoxy)-2 phényl]acétyl}-2 diphényl-7,7 perhydroisoindolol-4-(3aR,4S,7aR) sous la forme d'une meringue crème.

Spectre infra-rouge (KBr, bandes caractéristiques, $cm^{-1}$): 3080, 3050, 3020, 2940, 2870, 2815, 2765, 1635, 1600, 1490, 1455, 1445, 1245, 1065, 750, 730, 700.

Spectre RMN du proton (DMSO-$d_6$) à 433°K : 1,06 et 1,76 (2mt, 2x1H, $CH_2$ en 5) ; 2,27 (s, 6H, $N(CH_3)_2$) ; 3,9 (d, J=11, 1H, 1H du $CH_2$ en 3) ; 6,8 à 7,5 (mt, 14H, aromatiques)

Une solution de 100 g d'acide hydroxy-2 phénylacétique, 75 $cm^3$ d'alcool benzylique et 0,5 g d'acide paratoluène sulfonique dans 1400 $cm^3$ de toluène est chauffée au reflux pendant 2 heures en éliminant l'eau formée. Après refroidissement, traitement par 3 g de noir animal et filtration, le mélange réactionnel est concentré à 150 $cm^3$ et additionné de 300 $cm^3$ d'oxyde d'isopropyle. Les cristaux obtenus par refroidissement à 0°C sont essorés lavés et séchés pour donner 82,5 g d'hydroxy-2 phénylacétate de benzyle. A une solution 153 g de cet ester dans un mélange de 500 $cm^3$ de dibromo-1,3 propane et 2500 $cm^3$ d'acétonitrile on ajoute 174 g de carbonate de potassium et l'on chauffe le mélange 17 heures au reflux. Le mélange réactionnel est refroidi, filtré et concentré à sec sous pression réduite (2,7 kPa). Le résidu est repris par 500 $cm^3$ d'acétate d'éthyle et la phase organique est lavée par 2 fois par 400 $cm^3$ d'eau et 2 fois par 250 $cm^3$ de solution saturée de chlorure de sodium puis séchée et concentrée à sec sous pression réduite (2,7 kPa). Le résidu est chromatographié sur une colonne de gel de silice (granulométrie 0,2-0,063 mm, diamètre 9 cm, hauteur 55 cm) en éluant par un mélange de cyclohexane et d'acétate d'éthyle (95/5 en volumes) et en recueillant des fractions de 500 $cm^3$. Les fractions 12 à 18 sont réunies et concentrées à sec sous pression réduite (2,7 kPa) pour donner 90 g de (bromo-3 propoxy) -2 phénylacétate de benzyle sous la forme d'une huile Jaune. Une solution de 40 g de ce produit dans 500 $cm^3$ d'acétonitrile est chauffée en autoclave avec 27 g d'iodure de sodium et 90 g de diméthylamine pendant 16 heures à 80°C. Le mélange réactionnel est refroidi, filtré et concentré à sec sous pression réduite (2,7 kPa). Le résidu est purifié par passage acide-base pour donner 29,3 g de (diméthylamino-3 propoxy)-2 phénylacétate de benzyle sous la forme d'une huile jaune. L'hydrogènation de cet ester à pression ordinaire à 40°C dans l'acétate d'éthyle en présence d'hydroxyde de palladium puis cristallisation dans l'acétate d'éthyle conduisent à 17,5 g d'acide (diméthylamino-3 propoxy)-2 phénylacétique sous la forme de cristaux blanc fondant à 98 °C.

<u>Exemple d'utilisation 15</u>

A une solution de 0,41 g de tétrafluoroborate de (méthoxy-2 phényl)-acétimidate d'éthyle dans 10 $cm^3$ de dichlorométhane sec, on ajoute une solution de 0,6 g de chlorhydrate de diphényl-4,4 fluoro-7 perhydroisoindole-(3aR,7R,7aR) et de 0,51 $cm^3$ de triéthylamine dans 10 $cm^3$ de dichlorométhane sec. Le mélange réactionnel est chauffé 3 heures au reflux. Il est ensuite traité, après retour à température ambiante, par 5 $cm^3$ d'une solution aqueuse de carbonate de potassium à 10%; la phase organique est lavée par 10 $cm^3$ d'eau distillée, séchée sur sulfate de magnésium et concentrée à sec sous pression réduite (2,7 kPa). Le résidu est chromatographié sur colonne de gel de silice (granulométrie 0,04-0,06 mm, diamètre 2 cm, hauteur 20 cm), en éluant sous une pression de 0,6 bar d'azote par un mélange d'acétate d'éthyle, d'acide acétique et d'eau (15/1/1 en volumes) et en recueillant des fractions de 25 $cm^3$. Les fractions 24 à 38 sont réunies et concentrées à sec sous pression réduite (2,7 kPa). Le résidu est repris par 40 $cm^3$ de dichlorométhane, lavé par 10 $cm^3$ de solution aqueuse saturée de carbonate de potassium, puis par 10 $cm^3$ de solution aqueuse saturée de chlorure de sodium. La phase organique est séchée sur sulfate de magnésium et concentrée à sec sous pression réduite (2,7 kPa). Le résidu est cristallisé dans l'oxyde d'isopropyle. Les cristaux sont essorés et séchés. On obtient 0,18 g de diphényl-4,4 fluoro-7 [imino-1 (méthoxy-2 phényl)-2 éthyl]-2 perhydro-isoindole-(3aRS,7R,7aR) sous la forme de cristaux blancs fondant à 184°C avec décomposition.

Exemple d'utilisation 16

A une solution de 2 g de diphényl-7,7 perhydro-isoindolol-4-(3aR,4S,7aR) dans 30 $cm^3$ de dichlorométhane sec, on ajoute une solution de 1,56 g de tétrafluoroborate de (méthoxy-2 phényl)-acétimidate d'éthyle et de 0,96 $cm^3$ de triéthylamine dans 20 $cm^3$ de dichloroéthane sec, puis on porte le mélange réactionnel au reflux pendant 2 heures. On ajoute ensuite 10 $cm^3$ d'une solution aqueuse à 10% de carbonate de potassium, on décante, puis on lave la phase organique par 20 $cm^3$ d'eau, on sèche sur sulfate de magnésium, on filtre et concentre à sec sous pression réduite (2,7 kPa). Le résidu est chromatographié sur colonne d'alumine (diamètre 3,6 cm, hauteur 31 cm), en éluant sous une pression de 0,1 bar d'azote par un mélange de dichlorométhane et de méthanol (95/5 en volumes) et en recueillant des fractions de 50 $cm^3$. Les fractions 5 à 30 sont réunies et concentrées à sec sous pression réduite (2,7 kPa). Le solide obtenu est lavé à l'oxyde d'isopropyle, essoré et séché. On obtient 1,4 g de diphényl-7,7 [imino-1 (méthoxy-2 phényl)-2 éthyl]-2 perhydroisoindolol-4-(3aR,4S,7aR) sous la forme de cristaux blancs, fondant à 105°C avec décomposition.

## Revendications

**1 -** Nouveau dérivé de perhydroisoindole caractérisé en ce qu'il répond à la formule générale :

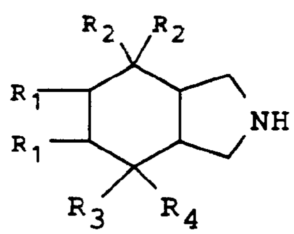

dans laquelle

- les radicaux $R_1$ sont identiques et représentent des atomes d'hydrogène ou forment ensemble une liaison,
- les symboles $R_2$ sont identiques et représentent des radicaux phényle éventuellement substitués par un atome d'halogène ou par un radical méthyle en position 2 ou 3,
- le symbole $R_3$ représente un atome d'halogène ou un radical hydroxy et
- le symbole $R_4$ représente un atome d'hydrogène ou simultanément à $R_3$ représente un atome d'halogène,

sous ses formes stéréoisomères ou leurs mélanges, ainsi que ses sels.

**2 -** Un dérivé de perhydroisoindole selon la revendication 1, caractérisé en ce que :

- les radicaux $R_1$ sont des atomes d'hydrogène,
- les symboles $R_2$ sont identiques et représentent des radicaux phényle,
- le symbole $R_3$ représente un atome de fluor ou de chlore ou un radical hydroxy et
- le symbole $R_4$ représente un atome d'hydrogène ou simultanément à $R_3$ représente un atome de fluor,

sous ses formes stéréoisomères ou leurs mélanges, ainsi que ses sels.

**3 -** Le diphényl-7,7 perhydroisoindol-4, sous ses formes stéréoisomères ou leurs mélanges, ainsi que ses sels.

**4 -** Le diphényl-4,4 fluoro-7 perhydroisoindole, sous ses formes stéréoisomères ou leurs mélanges, ainsi que ses sels.

**5** - Le diphényl-4,4 difluoro-7,7 perhydroisoindole, sous ses formes stéreoisomères ou leurs mélanges, ainsi que ses sels.

**6** - Le chloro-7 diphényl-4,4 perhydroisoindole, sous ses formes stéreoisomères ou leurs mélanges, ainsi que ses sels.

**7** - Procédé de préparation d'un nouveau dérivé de per-hydroisoindole selon la revendication 1 pour lequel $R_3$ est un atome d'halogène et $R_4$ est un atome d'hydrogène ou d'halogène, caractérisé en ce que l'on traite par un agent d'halogénation un dérivé de l'isoindole de formule générale :

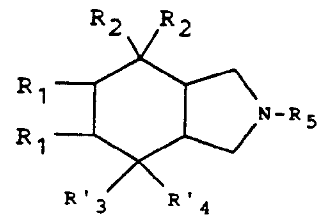

dans laquelle $R_1$ et $R_2$ sont définis comme précédemment dans la revendication 1, $R_5$ est un radical protecteur et, $R'_3$ est un radical hydroxy et $R'_5$ est un atome d'hydrogène si l'on veut obtenir un dérivé monohalogéné, ou $R'_3$ et $R'_4$ forment ensemble un radical oxo si l'on veut obtenir un dérivé dihalogéné, puis élimine le radical protecteur $R_5$ et transforme éventuellement le produit obtenu en un sel.

**8** - Procédé de préparation d'un nouveau dérivé de perhydroisoindole selon la revendication 1 pour lequel $R_3$ est un atome d'halogène et $R_4$ est un atome d'hydrogène, caractérisé en ce que l'on traite par un agent d'halogénation un dérivé de l'isoindole de formule générale :

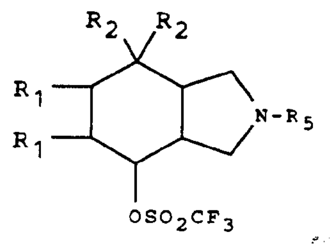

dans laquelle $R_1$, $R_2$ et $R_5$ sont définis comme dans la revendication 2, puis élimine le radical protecteur $R_5$ et transforme éventuellement le produit obtenu en un sel.

**9** - Procédé de préparation d'un dérivé de perhydroisoindole selon la revendication 1 pour lequel $R_3$ est un radical hydroxy et $R_4$ est un atome d'hydrogène, caractérisé en ce que l'on réduit un dérivé de perhydroisoindolone de formule générale :

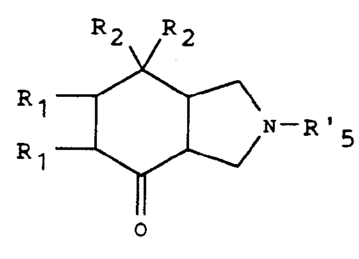

dans laquelle $R_1$ et $R_2$ sont définis comme dans la revendication 1 et $R'_5$ est défini comme $R_5$ ou représente un atome d'hydrogène, puis sépare éventuellement les isomères axial et équatorial et transforme éventuellement le produit obtenu en un sel.

**10** - Procédé de préparation d'un dérivé de perhydroisoindole selon la revendication 1 pour lequel $R_3$ est un radical hydroxy et $R_4$ est un atome d'hydrogène, caractérisé en ce que l'on élimine le radical protecteur du dérivé de perhydroisoindole de formule générale :

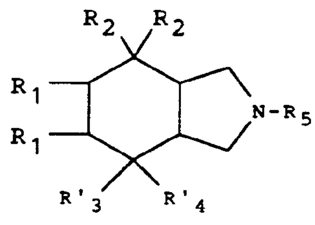

dans laquelle $R_1$ et $R_2$ sont définis comme précédemment dans la revendication 1, $R_5$ est un radical protecteur, $R'_3$ est un radical hydroxy et $R'_4$ est un atome d'hydrogène, puis transforme éventuellement le produit obtenu en un sel.

**11-** Utilisation d'un dérivé de l'isoindole selon la revendication 1 pour la préparation d'un produit de formule générale :

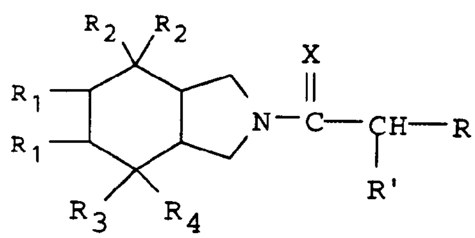

dans laquelle

- les radicaux $R_1$ sont identiques et représentent des atomes d'hydrogène ou forment ensemble une liaison,
- les symboles $R_2$ sont identiques et représentent des radicaux phényle éventuellement substitués par un atome d'halogène ou par un radical méthyle en position 2 ou 3,
- le symbole X représente un atome d'oxygène, ou un radical NH,
- le symbole R représente un radical phényle éventuellement substitué par un ou plusieurs atomes d'halogène ou radicaux hydroxy, alcoyle pouvant être éventuellement substitués (par des atomes d'halogène ou des radicaux amino, alcoylamino ou dialcoylamino) alcoyloxy ou alcoylthio pouvant être éventuellement substitués (par des radicaux hydroxy, amino, alcoylamino ou dialcoylamino éventuellement substitués (par des radicaux phényle, hydroxy ou amino), ou dialcoylamino dont les parties alcoyle forment avec l'atome d'azote auquel elles sont rattachées, un hétérocycle à 5 à 6 chaînons pouvant contenir un autre hétéroatome choisi parmi l'oxygène, le soufre ou l'azote, éventuellement substitué par un radical alcoyle, hydroxy, hydroxyalcoyle)], ou substitué par des radicaux amino, alcoylamino, dialcoylamino dont les parties alcoyle peuvent former avec l'atome d'azote auquel elles sont rattachées, un hétérocycle tel que défini ci-dessus, ou représente un radical cyclohexadiènyle, naphtyle ou hétérocyclyle mono ou polycyclique, saturé ou insaturé contenant 5 à 9 atomes de carbone et un ou plusieurs hétéroatomes choisis parmi l'oxygène, l'azote ou le soufre,
- le symbole R' représente un atome d'hydrogène ou d'halogène ou un radical hydroxy, alcoyle, aminoalcoyle, alcoylaminoalcoyle, dialcoylaminoalcoyle, alcoyloxy, alcoylthio, acyloxy, carboxy, alcoyloxycarbonyle, dialcoylaminoalcoyloxycarbonyle, benzyloxycarbonyle, amino, acylamino ou alcoyloxycarbonylamino,
- le symbole $R_3$ représente un atome d'halogène ou un radical hydroxy et
- le symbole $R_4$ représente un atome d'hydrogène ou simultanément à $R_3$, représente un atome d'halogène,

les radicaux alcoyle et acyle cités ci-dessus étant droits ou ramifiés et contenant 1 à 4 atomes de carbone, sous leurs formes stéréoisomères ou leurs mélanges, ainsi que leurs sels lorsqu'ils existent.

Office européen des brevets

# RAPPORT DE RECHERCHE EUROPEENNE

Numero de la demande

EP 92 40 1330

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A,D | US-A-4 042 707 (W.C. RIPKA)<br>* colonne 31, tableau I *<br>--- | 1 | C07D209/44 |
| A | EP-A-0 068 822 (ROHM AND HAAS CO.)<br>* abrégé *<br>--- | 1 | |
| A | AU-B-502 760 (SCIENCE UNION ET CIE.)<br>* revendications 1,2 *<br>--- | 1 | |
| A | EP-A-0 359 172 (SHIONOGI AND CO., LTD.)<br>* page 15, composé (II-11) *<br>--- | 1 | |
| A | EP-A-0 058 567 (WARNER-LAMBERT CO.)<br>* abrégé *<br>--- | 11 | |
| A | EP-A-0 093 805 (WARNER-LAMBERT CO.)<br>* abrégé *<br>----- | 11 | |
| | | | **DOMAINES TECHNIQUES RECHERCHES (Int. Cl.5 )** |
| | | | C07D |

Le présent rapport a été établi pour toutes les revendications

| Lieu de la recherche | Date d'achèvement de la recherche | Examinateur |
|---|---|---|
| BERLIN | 21 AOUT 1992 | CHRISTIAN HASS |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)